# EUROPEAN PATENT APPLICATION

(11) **EP 1 403 779 A1**
(43) Date of publication of application: **31.03.2004**
(21) Application number: 02743697.1
(22) Date of filing: 24.06.2002
(51) Int. Cl.: G06F 17/30, G06F 12/00

(54) **STRUCTURED DATA PROCESSING APPARATUS**

(30) Priority: 22.06.2001 JP 2001189631
(71) Applicant: Celestar Lexico-Sciences, Inc., Chiba-shi, Chiba 261-8501 (JP)
(72) Inventor: NITTA, Kiyoshi, c/o Celestar Lexico-Sciences, Inc., Chiba-shi, Chiba 261-8501 (JP); UEMURA, Yasuo, c/o Celestar Lexico-Sciences, Inc., Chiba-shi, Chiba 261-8501 (JP)
(74) Representative: Leson, Thomas Johannes Alois, Dipl.-Ing.
(86) International application number: PCT/JP2002/006288
(87) International publication number: WO 2003/001409

(57) **Abstract**

A structured data processing apparatus (100) includes a control unit (102) such as a CPU for overall controlling the structured data processing apparatus (100), a communication control interface unit (104) connected to a communication apparatus (not depicted) such as a router connected to a communication line, and an input/output control interface unit (108) connected to an input device (112) and an output device (114), and a storage unit (106) for storing various databases and tables. These components are communicably connected via a communication path.

## Description

### TECHNICAL FIELD

The present invention relates to a structured data processing apparatus, a structured data processing method, a computer program, and a recording medium capable of efficiently processing structured data in various formats defined by schema languages in various formats.

### BACKGROUND ART

Large-scale sequence information databases of base and amino acid, and bibliographic information databases are fundamental databases used in the field of bio-informatics. For example, "GenBank" is an existing sequence information database and "PubMed" is an existing bibliographic information database (see http://www.ncbi.nlm.nih.gov/Genbank).

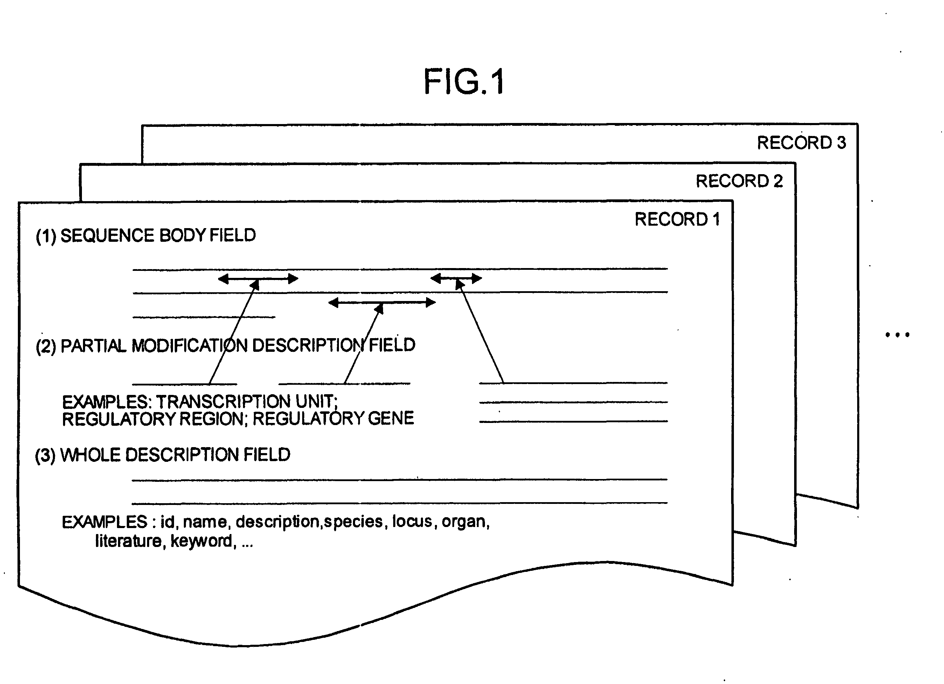

Fig. 1 is an illustration of one example of the basic data structure of the sequence information database of base sequences of genes or amino acid sequences of proteins.

As illustrated in Fig. 1, the data structure of each piece of the sequence information stored in the sequence information database normally consists of three fields: (1) a field that stores a sequence body, (2) a partial modification description field that stores annotation information on a part of the sequence, and (3) a whole description field that stores annotation information on the whole sequence.

The sequence body field (1) consists of a base sequence or an amino acid sequence. The base sequence is a one-dimensional sequence of four types of bases (ACGT) that constitute the chromosome of a biological cell. If the base sequence acts as a gene, a specific protein is produced from specific sequence information of the base sequence. The amino acid sequence is a one-dimensional sequence of 20 types of amino acids that constitute the protein.

The partial modification description field (2) stores annotation information about a part of the sequence body, such as knowledge (for example, physical properties and structure information) that is obtained through experiment or analysis. Some sequences include no such annotation information whereas some include more than one partial modification description field.

The whole description field (3) stores information on the whole sequence. For example, the whole description field consists of data on a classification ID, a common name, an explanation in a natural language, a biological species, a position on a chromosome, an organ (if the information is expression data), references to relevant scientific literatures, and a keyword of the whole sequence.

These pieces of sequence information stored in the database tend to differ in fields to be filled per record, and the number of repetitions per record. Therefore, these pieces of sequence information are often distributed in a certain text format or a structured description format such as XML (Extensible Markup Language).

Examples of existing structured description languages used in the field of bioinformatics include "Abstract Syntax Notation 1 (ASN.1)" (http://www.ncbi.nlm.nih.gov/Sitemap/Summary/asn1.html, James M. Ostell, "Integrated Access to Heterogeneous Data from NCBI", pp. 730-736, IEEE Engineering in Medicine and Biology, Nov/Dec, 1995), and XML-based "Bio Sequence Markup Language (BSML)" (http://www.labbook.com/fag/bsml.asp), "The BIOpolymer Markup Language (BioML)" (http://www.bioml.com/BIOML/index.html) and "Genome Annotation Markup Elements (GAME)" (http://www.bioxml.org/Projects/game/).

The sequence data is large in scale (for example, GenBank has records on the order of ten million). To ensure an efficient search processing, the data is converted and stored in a database system which uses a relational database (RDB).

The conventional system has, however, the following two problems.
(1) The system does not have a high extensibility required to store data of various structured description formats.
(2) The system cannot efficiency store and use data.
These two problems are explained in detail below.

The high extensibility related to the data description formats as explained in Problem (1) is important particularly in the field of bioinformatics (hereinafter, "bioinformatics field"). All pieces of information to be stored in the bioinformatics field are not expressed in the existing structured description language such as XML, BSML or BioML only. As the research in the bioinformatics field advances, a set of definition information (schema) on the information to be stored keeps changing. For example, if a new experimental means is developed, a field that stores the result of the experiment and a schema used to define information thereon are added.

In addition, a repetitive construct is often introduced so as to store the same fact in a plurality of expressions. If so, data described in the existing format should be converted into data in a new form. As a result, there is a need to develop a conversion program and consequently, there is an additional conversion processing cost.

Further, if information on a plurality of interacting protein regions is to be included in protein records without changing frameworks, it is necessary to store the same information in two different records synchronously with each other. This results in the use of multiple storage regions, which in turn, causes management problems like implementing functions such as storage and correction functions.

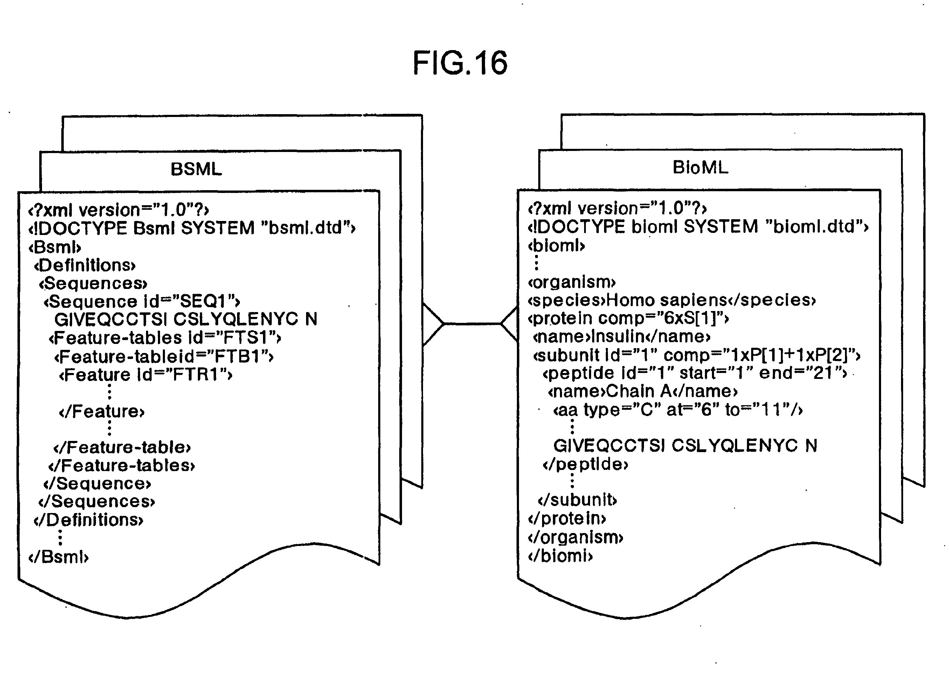

Fig. 16 illustrates the structural difference between structured data described in BSML and that described in BioML. Both BSML and BioML are normally used in the bioinformatics field.

As explained, there still exist structured description formats described in a plurality of types of structured description languages. To reuse existing software resources, it is necessary to easily convert the data into existing formats. As illustrated in Fig: 16, there is a structural difference in the format of the partial modification description field particularly between BSML and BioML. In BioML, a part of partial modification description related to the structure of a protein is embedded in the tree structure of an XML document. In BSML, the entire partial modification description is given differently as a combination of sequence position information. To efficiently convert data into data of such different formats, the expression ability of the storage structure is required to be flexible enough.

Problem (2) is about efficiency when the flexible data that can solve the Problem (1) is used.

Since the RDB technique has been long put to practical use, it is highly reliable and can be used with excellent processing efficiency for large-scale data. However, according to the RDB technique, a data model is designed on the premise that the schema of data to be handled in a target domain is static. The more complex the data structure is, the higher the degree of fixing the schema becomes. Therefore, the construction of a system having high extensibility as required to solve the Problem (1) is not originally assumed, thus bringing about the efficiency problem.

If the RDB is unavailable, data is stored in plain text files of the most flexible storage type. If plain text files are used, however, efficiency of searching and fetching large-scale data is low. Particularly in the bioinformatics field, large-scale analyses are often conducted on these pieces of large-scale data. As a result, efficiency required for the handling of each record is higher than that required for a business document processing or a transaction processing performed by an end user.

It is, therefore, an object of the present invention to provide a structured data processing apparatus, a structure data processing method, a program, and a recording medium capable of storing large-scale data such as sequence information including the base sequence of a gene and an amino acid sequence of a protein, in a format having high extensibility, and efficiently storing and using the data.

### DISCLOSURE OF THE INVENTION

A structured data processing apparatus according to the present invention comprises: a structure data acquisition unit which acquires structured data described in a structured description language, and schema data defining a structure of the structured data; a format conversion unit which converts the structured data and the schema data acquired by the structured data acquisition unit based on schema format conversion instruction information; a structured data registration unit which registers the structured data and the schema data converted by the format conversion unit in a database; an analysis tool registration unit which registers a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and an analysis tool start unit which converts the structured data and the schema data registered in the database according to the schema resource definition information corresponding to the tool program, and which inputs the converted structured data and schema data to the tool program if the tool program is started.

According to this processing apparatus, structured data described in a structured description language and schema data defining a structure of the structured data are acquired, the structured data and the schema data thus acquired are converted based on schema format conversion instruction information, the structured data and the schema data thus converted are registered in a database, a tool program for accessing the database, in which the structured data and the schema data are registered by the structured data registration unit, to conduct a data processing, and schema resource definition information which defines resources of a schema of the structured data input to the tool program are registered so that the tool program corresponds to the schema resource definition information, and the structured data and the schema data registered in the database are dynamically converted according to the schema resource definition information corresponding to the tool program and the converted structured data and schema data are input to the tool program if the tool program is started. Therefore, the formats of the acquired data described in different structured languages and schema languages can be converted into formats set in advance or set at need.

Further, it is possible to facilitate matching the data acquired from various external databases and ensure high extensibility related to data description formats. This consequently can facilitate accessing to the external databases corresponding to various data description formats. That is, it is possible to manage an internal database in the format of the uniform, specific structured description language (for example, BSML or BioML), so that database utilization efficiency and the like can be significantly improved.

In addition, even if a new resource (for example, an XML element) is added to the schema, the format of the schema can be easily converted into an added form.

Moreover, even if an item is added at an appropriate time by each analysis tool and the added item is used in a processing by a later analysis tool processing, it is possible to easily ensure the extensibility of each data to be used without changing the specification of the analysis tool. It is also possible to convert the format of a shared part database by batch processing.

The next invention provides a structured data processing apparatus, wherein the structured description language is one of XML, SGML (Standard Generalized Markup Language), BioML, BSML, ASN.1, GAME, structured description languages extended from these six languages, and structured description languages equivalent in description ability to these six languages.

This invention presents an example of the structured description language more concretely. According to this processing apparatus, the structured description language is one of XML, SGML, BioML, BSML, ASN.1, GAME, structured description languages extended from these six languages, and structured description languages equivalent in description ability to the six languages. Therefore, this processing apparatus can efficiently convert the structured data described in these structured description language normally used in the bioinformatics field.

The next invention provides a structured data processing apparatus according to the above structured data processing apparatus, wherein that the schema data is data described in one of DTD (Data Type Definition), XML schema, RELAX (Regular Language Description for XML), schema languages extended from these three languages, and schema languages equivalent in description ability to these three languages.

This invention presents an example of the schema data more concretely. According to this processing apparatus, the schema data is data described in one of DTD, XML schema, RELAX, schema languages extended from these three languages, and schema languages equivalent in description ability to these three languages. Therefore, this processing apparatus can efficiently convert the schema data described in these schema languages normally used in the bioinformatics field.

The next invention provides a structured data processing apparatus according to the above structured data processing apparatus, wherein the schema format conversion instruction information and the schema resource definition information are data described in one of the XSL (Extensible Stylesheet Language), the language extended from the XSL, and tree structure conversion languages equal in description ability to the XSL and the XSL extended language.

This invention presents an example of the schema format conversion instruction information and the schema resource definition information more concretely. According to this processing apparatus, the schema format conversion instruction information and the schema resource definition information are data described in one of the XSL, the language extended from the XSL, and tree structure conversion languages equal in description ability to the XSL and the XSL extended language. Therefore, the processing apparatus can efficiently convert the structured data and the schema data based on the schema format conversion instruction information and the schema resource definition information described in these schema conversion description languages normally used in the bioinformatics field.

The next invention provides a structured data processing apparatus according to the above structured data processing apparatus, wherein the structured data includes an element about at least one of sequence information, which includes one of or both of base sequences and amino acid sequences, and literature information.

This invention presents an example of the schema format conversion instruction information and the schema resource definition information more concretely. According to this processing apparatus, the structured data includes an element about at least one of sequence information, which includes one of or both of base sequences and amino acid sequences, and literature information. Therefore, the processing apparatus can acquire sequence information registered in GenBank or the like or literature information registered in PubMed or the like, and converts the format of the acquired information.

A structured data processing method according to the next invention comprises: a structure data acquisition step of acquiring structured data described in a structured description language, and schema data defining a structure of the structured data; a format conversion step of converting the structured data and the schema data acquired by the structured data acquisition unit based on schema format conversion instruction information; a structured data registration step of registering the structured data and the schema data converted by the format conversion unit in a database; an analysis tool registration step of registering a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and an analysis tool starting step of converting the structured data and the schema data registered in the database according to the schema resource definition information corresponding to the tool program, and inputting the converted structured data and schema data to the tool program if the tool program is started.

According to this method, structured data described in a structured description language and schema data defining a structure of the structured data are acquired, the structured data and the schema data thus acquired are converted based on schema format conversion instruction information, the structured data and the schema data thus converted are registered in a database, a tool program for accessing the database, in which the structured data and the schema data are registered by the structured data registration unit, to conduct a data processing, and schema resource definition information which defines resources of a schema of the structured data input to the tool program are registered so that the tool program corresponds to the schema resource definition information, and the structured data and the schema data registered in the database are dynamically converted according to the schema resource definition information corresponding to the tool program and the converted structured data and schema data are input to the tool program if the tool program is started. Therefore, the formats of the acquired data described in different structured languages and schema languages can be converted into formats set in advance or set at need.

Further, it is possible to facilitate matching the data acquired from various external databases and ensure high extensibility related to data description formats. This consequently can facilitate accessing to the external databases corresponding to various data description formats. That is, it is possible to manage an internal database in the format of the uniform, specific structured description language (for example, BSML or BioML), so that database utilization efficiency and the like can be significantly improved.

In addition, even if a new resource (for example, an XML element) is added to the schema, the format of the schema can be easily converted into an added form.

Moreover, even if an item is added at an appropriate time by each analysis tool and the added item is used in a processing by a later analysis tool processing, it is possible to easily ensure the extensibility of each data to be used without changing the specification of the analysis tool. It is also possible to convert the format of a shared part database by batch processing.

The next invention provides a structured data processing method according to the above structured data processing method, wherein the structured description language is one of XML, SGML, BioML, BSML, ASN.1, GAME, structured description languages extended from these six languages, and structured description languages equivalent in description ability to these six languages.

This invention presents an example of the structured description language more concretely. According to this method, the structured description language is one of XML, SGML, BioML, BSML, ASN.1, GAME, structured description languages extended from these six languages, and structured description languages equivalent in description ability to these six languages. Therefore, this method enables efficiently converting the structured data described in these structured description language normally used in the bioinformatics field.

The next invention provides a structured data processing method according to the above structured data processing method, wherein the schema data is data described in one of DTD, XML schema, RELAX, schema languages extended from these three languages, and schema languages equivalent in description ability to these three languages.

This invention presents an example of the schema data more concretely. According to this method, the schema data is data described in one of DTD, XML schema, RELAX, schema languages extended from these three languages, and schema languages. equivalent in description ability to these three languages. Therefore, this method enables efficiency converting the schema data described in these schema languages normally used in the bioinformatics field.

The next invention provides a structured data processing method according to the above structured data processing method, wherein the schema format conversion instruction information and the schema resource definition information are data described in one of the XSL, the language extended from the XSL, and tree structure conversion languages equal in description ability to the XSL and the XSL extended language.

This invention presents an example of the schema format conversion instruction information and the schema resource definition information more concretely. According to this method, the schema format conversion instruction information and the schema resource definition information are data described in one of the XSL, the language extended from the XSL, and tree structure conversion languages equal in description ability to the XSL and the XSL extended language. Therefore, the method enables efficiently converting the structured data and the schema data based on the schema format conversion instruction information and the schema resource definition information described in these schema conversion description languages normally used in the bioinformatics field.

The next invention provides a structured data processing method according to the above structured data processing method, wherein the structured data includes an element about at least one of sequence information, which includes one of or both of base sequences and amino acid sequences, and literature information.

This invention presents an example of the schema format conversion instruction information and the schema resource definition information more concretely. According to this method, the structured data includes an element about at least one of sequence information, which includes one of or both of base sequences and amino acid sequences, and literature information. Therefore, the method enables acquiring sequence information registered in GenBank or the like or literature information registered in PubMed or the like, and converting the format of the acquired information.

The next invention provides a program, which allows a computer to execute a structured data processing method, comprising: a structure data acquisition step of acquiring structured data described in a structured description language, and schema data defining a structure of the structured data; a format conversion step of converting the structured data and the schema data acquired by the structured data acquisition unit based on schema format conversion instruction information; a structured data registration step of registering the structured data and the schema data converted by the format conversion unit in a database; an analysis tool registration step of registering a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and an analysis tool starting step of converting the structured data and the schema data registered in the database according to the schema resource definition information corresponding to the tool program, and inputting the converted structured data and schema data to the tool program if the tool program is started.

According to this program, structured data described in a structured description language and schema data defining a structure of the structured data are acquired, the structured data and the schema data thus acquired are converted based on schema format conversion instruction information, the structured data and the schema data thus converted are registered in a database, a tool program for accessing the database, in which the structured data and the schema data are registered by the structured data registration unit, to conduct a data processing, and schema resource definition information which defines resources of a schema of the structured data input to the tool program are registered so that the tool program corresponds to the schema resource definition information, and the structured data and the schema data registered in the database are dynamically converted according to the schema resource definition information corresponding to the tool program and the converted structured data and schema data are input to the tool program if the tool program is started. Therefore, the formats of the acquired data described in different structured languages and schema languages can be converted into formats set in advance or set at need.

Further, it is possible to facilitate matching the data acquired from various external databases and ensure high extensibility related to data description formats. This consequently can facilitate accessing to the external databases corresponding to various data description formats. That is, it is possible to manage an internal database in the format of the uniform, specific structured description language (for example, BSML or BioML), so that database utilization efficiency and the like can be significantly improved.

In addition, even if a new resource (for example, an XML element) is added to the schema, the format of the schema can be easily converted into an added form.

Moreover, even if an item is added at an appropriate time by each analysis tool and the added item is used in a processing by a later analysis tool processing, it is possible to easily ensure the extensibility of each data to be used without changing the specification of the analysis tool. It is also possible to convert the format of a shared part database by batch processing.

The next invention provides a program according to the above program, wherein the structured description language is one of XML, SGML, BioML, BSML, ASN.1, GAME, structured description languages extended from these six languages, and structured description languages equivalent in description ability to these six languages.

This invention presents an example of the structured description language more concretely. According to this program, the structured description language is one of XML, SGML, BioML, BSML, ASN.1, GAME, structured description languages extended from these six languages, and structured description languages equivalent in description ability to these six languages. Therefore, the program enables efficiently converting the structured data described in these structured description language normally used in the bioinformatics field.

The next invention provides a program according to the above program, wherein the schema data is data described in one of DTD, XML schema, RELAX, schema languages extended from these three languages, and schema languages equivalent in description ability to these three languages.

This invention presents an example of the schema data more concretely. According to this program, the schema data is data described in one of DTD, XML schema, RELAX, schema languages extended from these three languages, and schema languages equivalent in description ability to these three languages. Therefore, the program enables efficiency converting the schema data described in these schema languages normally used in the bioinformatics field.

The next invention provides a program according to the above program, wherein the schema format conversion instruction information and the schema resource definition information are data described in one of the XSL, the language extended from the XSL, and tree structure conversion languages equal in description ability to the XSL and the XSL extended language.

This invention presents an example of the schema format conversion instruction information and the schema resource definition information more concretely. According to this program, the schema format conversion instruction information and the schema resource definition information are data described in one of the XSL, the language extended from the XSL, and tree structure conversion languages equal in description ability to the XSL and the XSL extended language. Therefore, the program enables efficiently converting the structured data and the schema data based on the schema format conversion instruction information and the schema resource definition information described in these schema conversion description languages normally used in the bioinformatics field.

The next invention provides a program according to the above program, wherein the structured data includes an element about at least one of sequence information, which includes one of or both of base sequences and amino acid sequences, and literature information.

This invention presents an example of the schema format conversion instruction information and the schema resource definition information more concretely. According to this program, the structured data includes an element about at least one of sequence information, which includes one of or both of base sequences and amino acid sequences, and literature information. Therefore, the program enables acquiring sequence information registered in GenBank or the like or literature information registered in PubMed or the like, and converting the format of the acquired information.

A recording medium according to the next invention records the program according to any one of the inventions.

According to this recording medium, a program recorded in the recording medium is read and executed by a computer, whereby the program can be realized using the computer. Therefore, the recording medium can attain the same advantages as those of the methods.

### BRIEF DESCRIPTION OF THE DRAWINGS

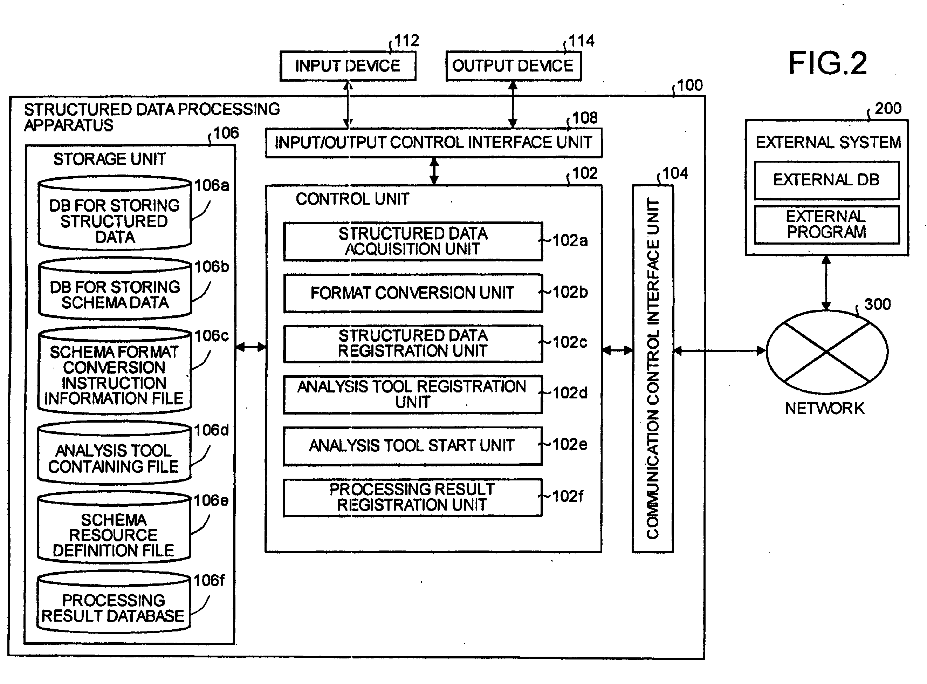

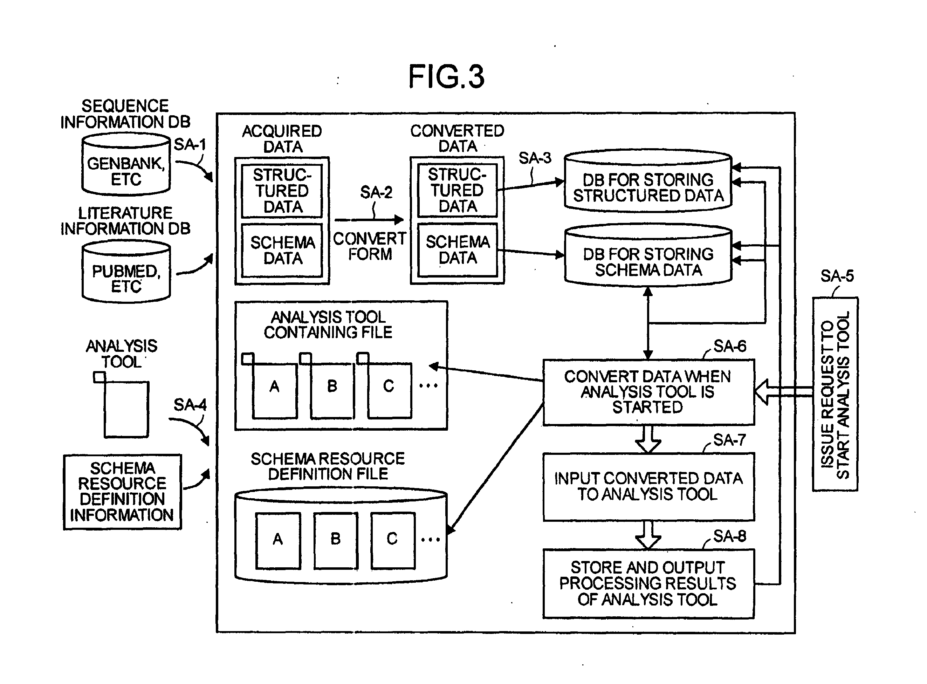

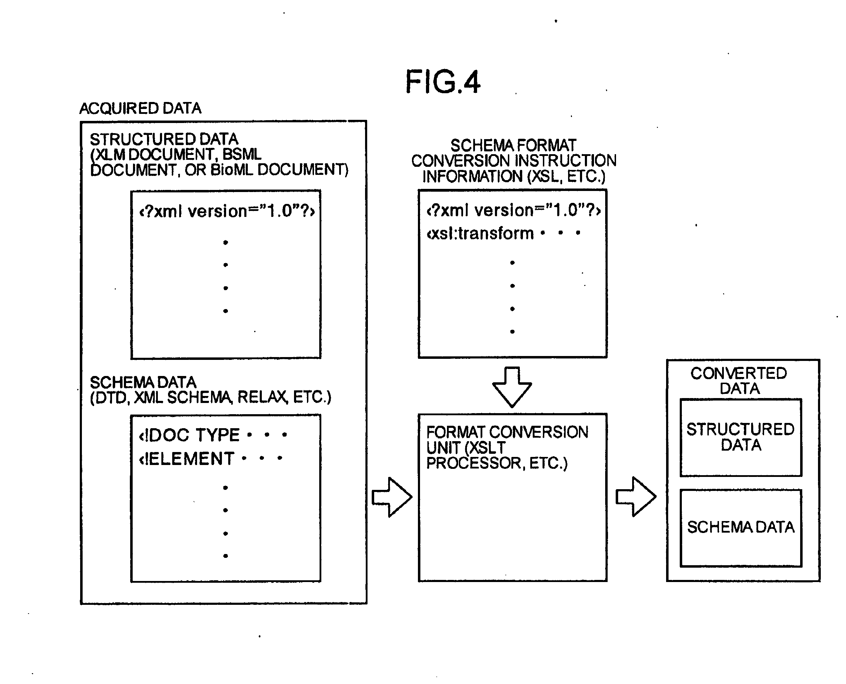

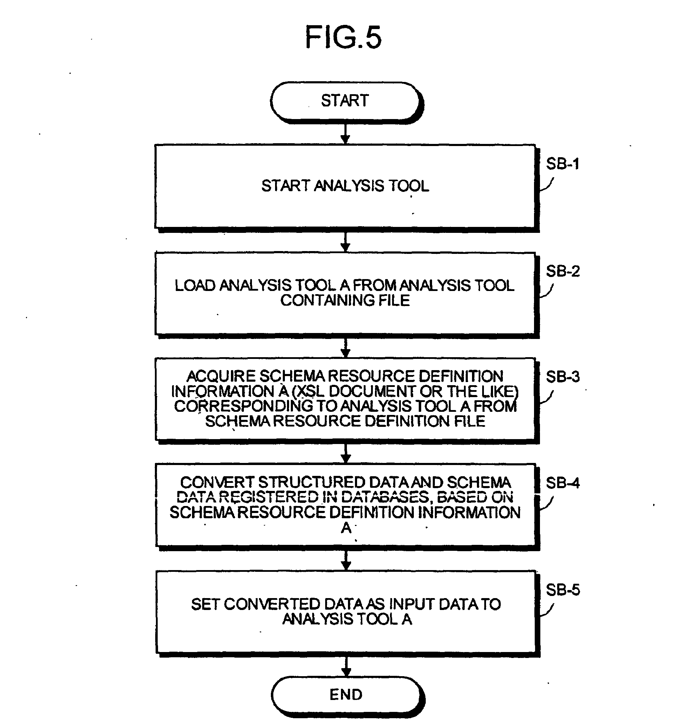

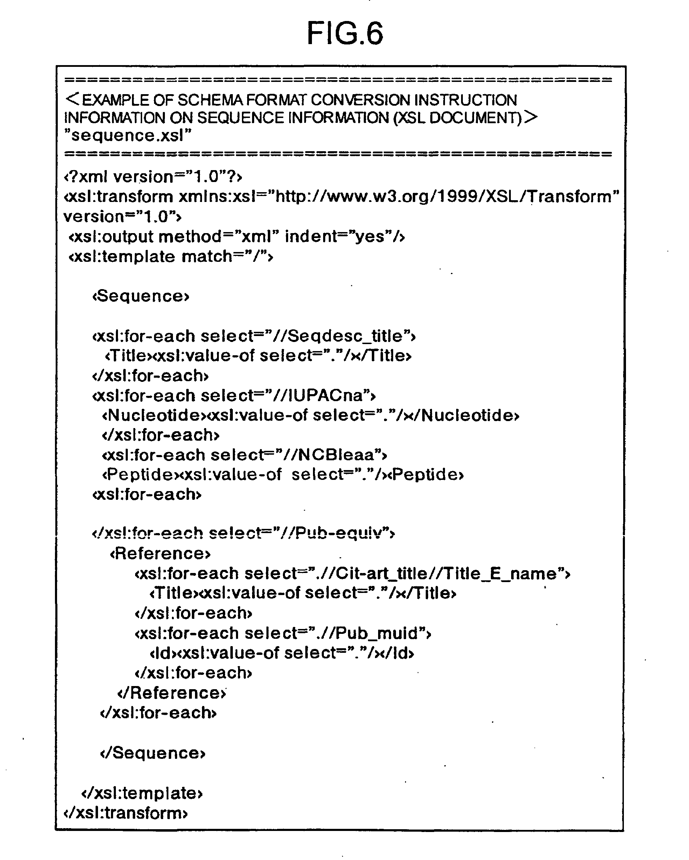

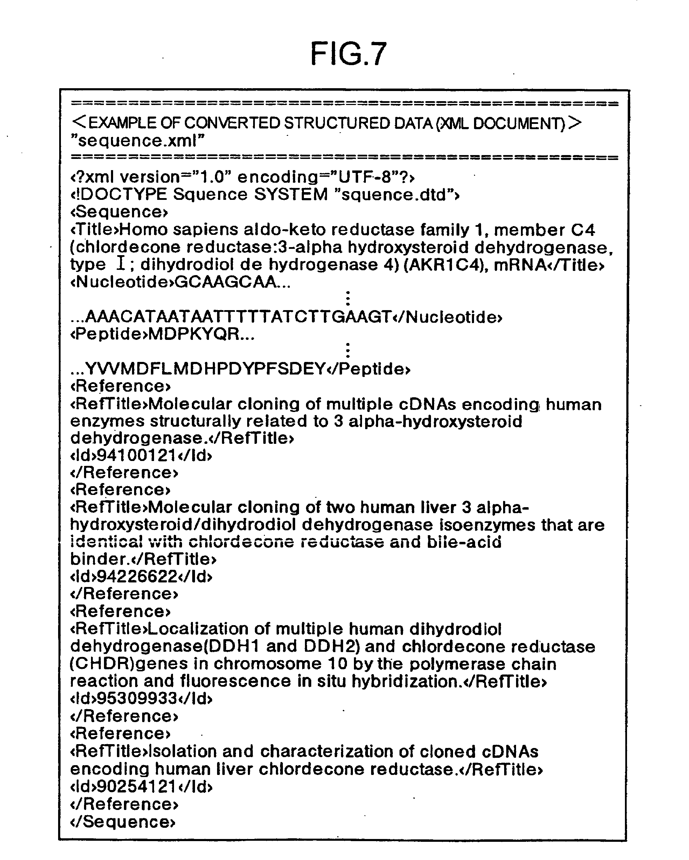

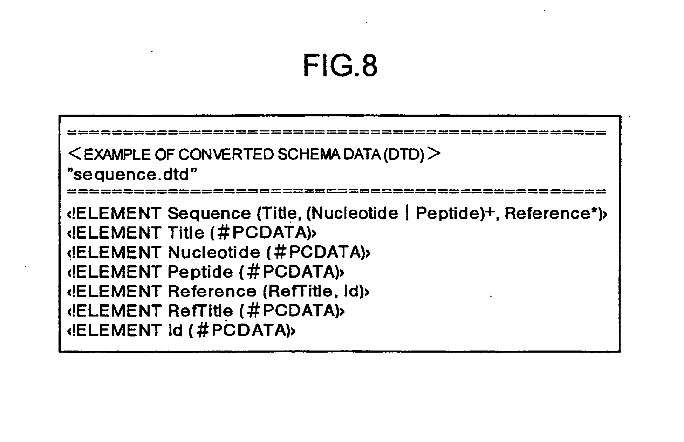

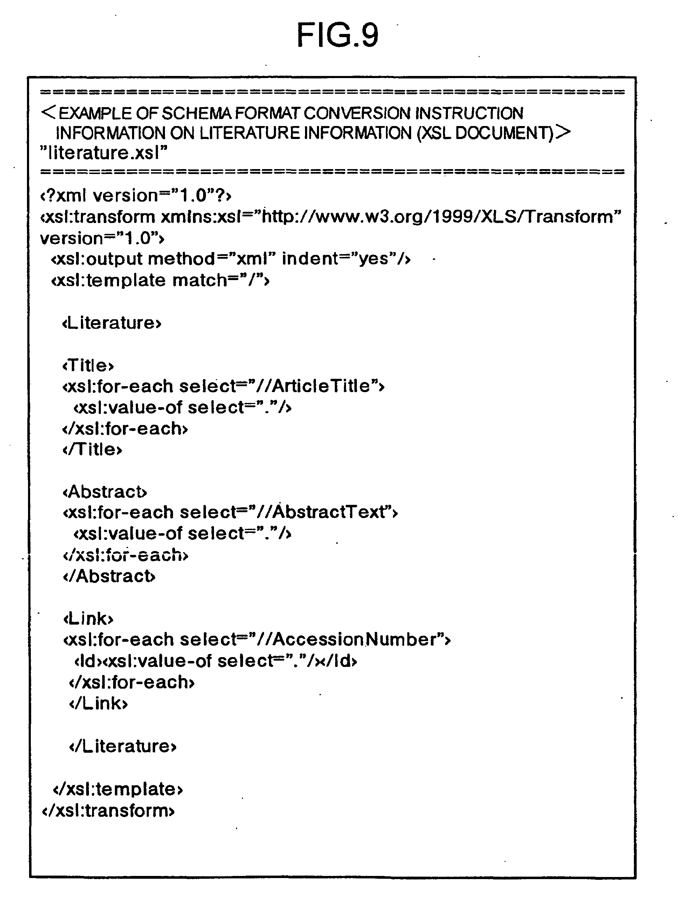

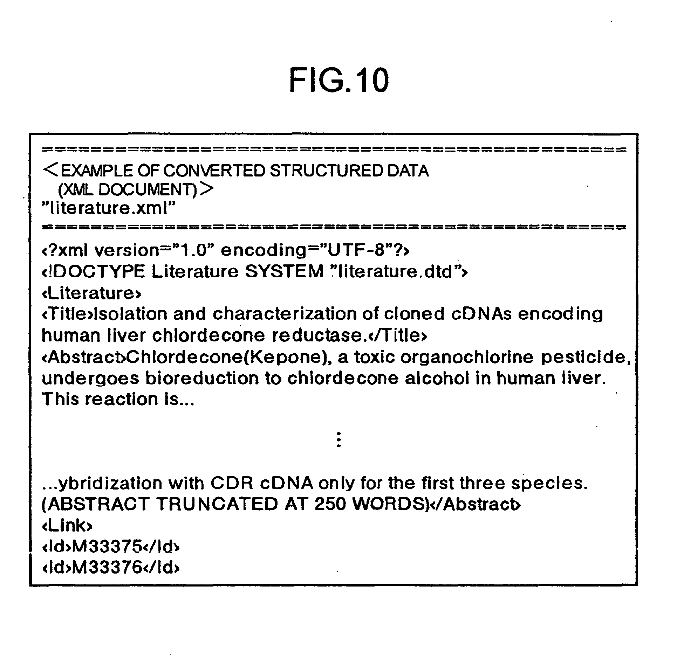

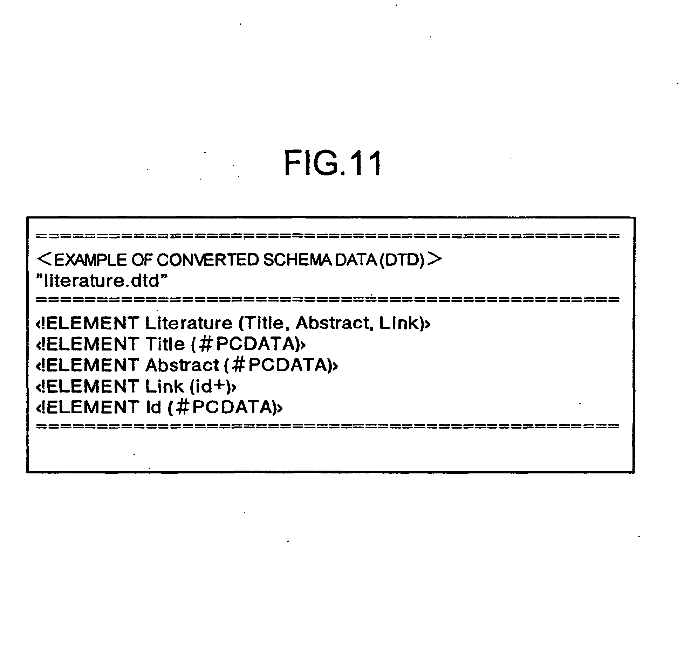

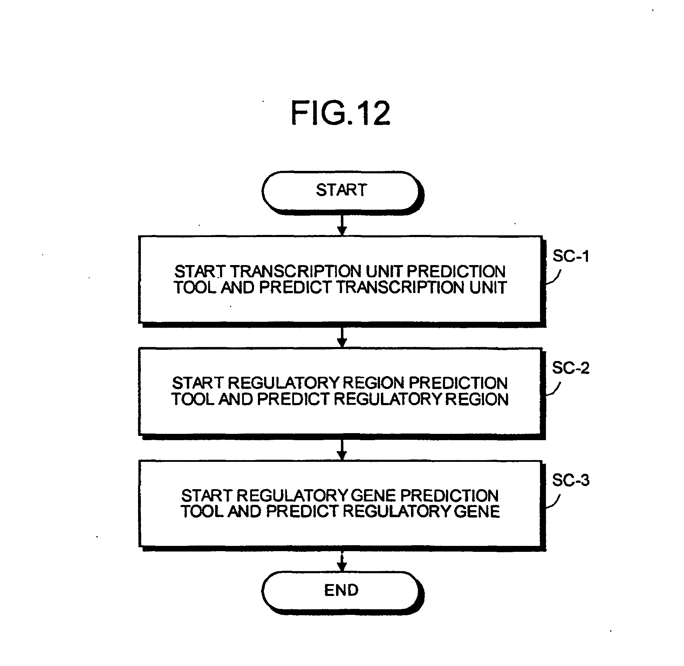

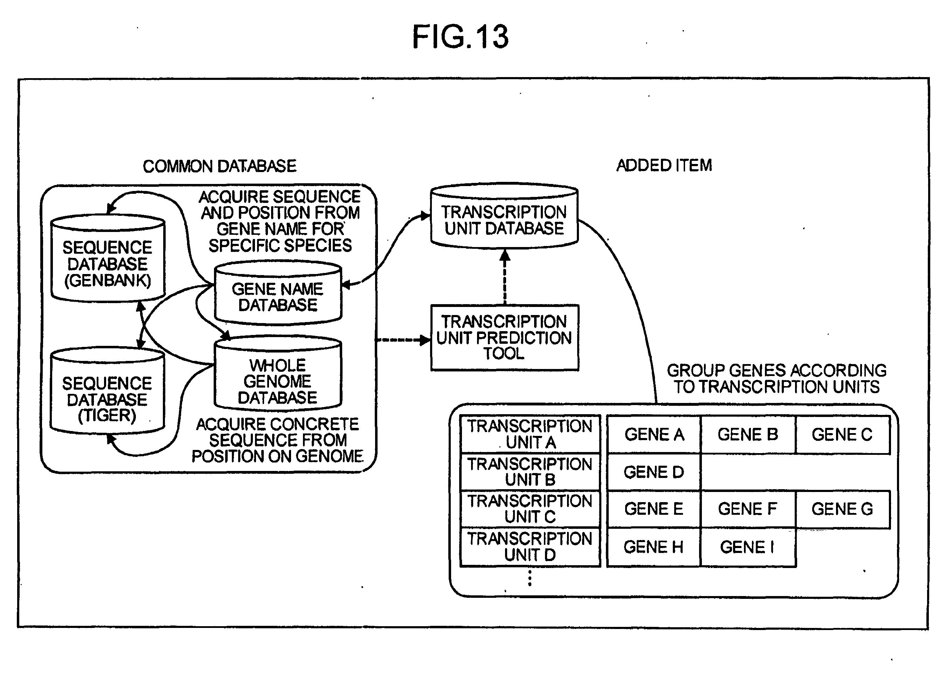

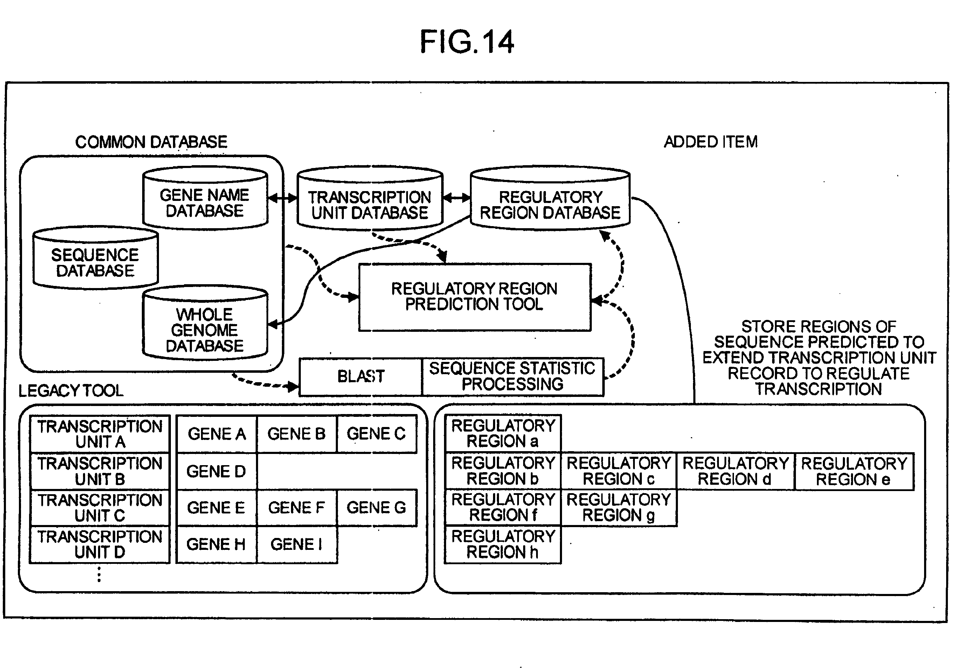

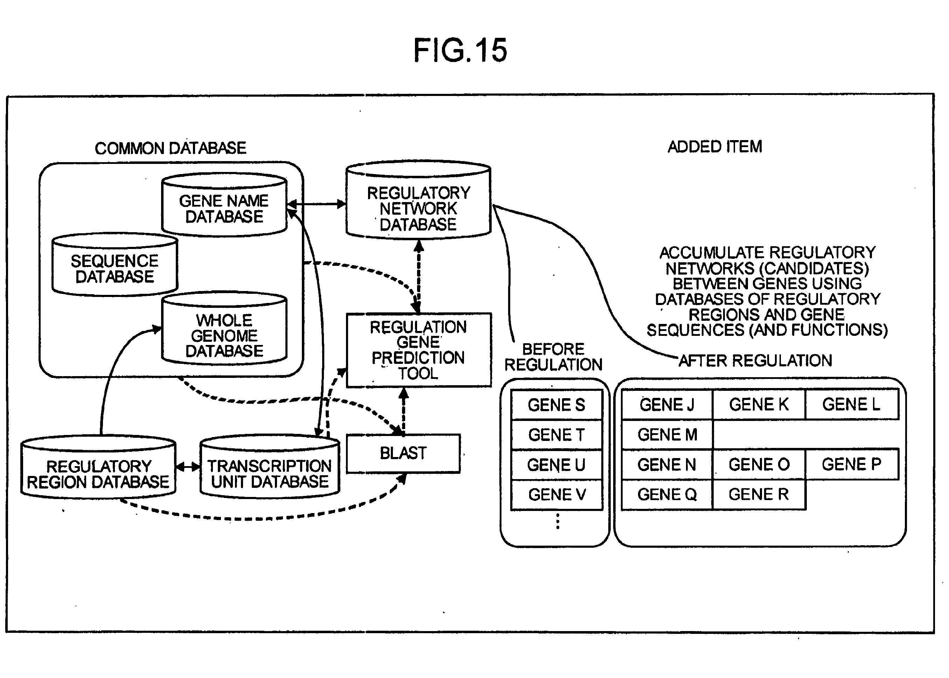

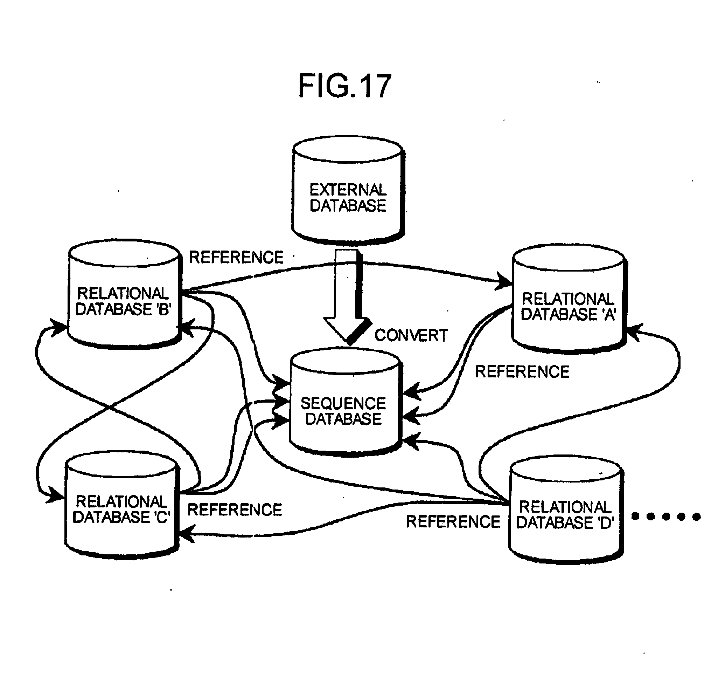

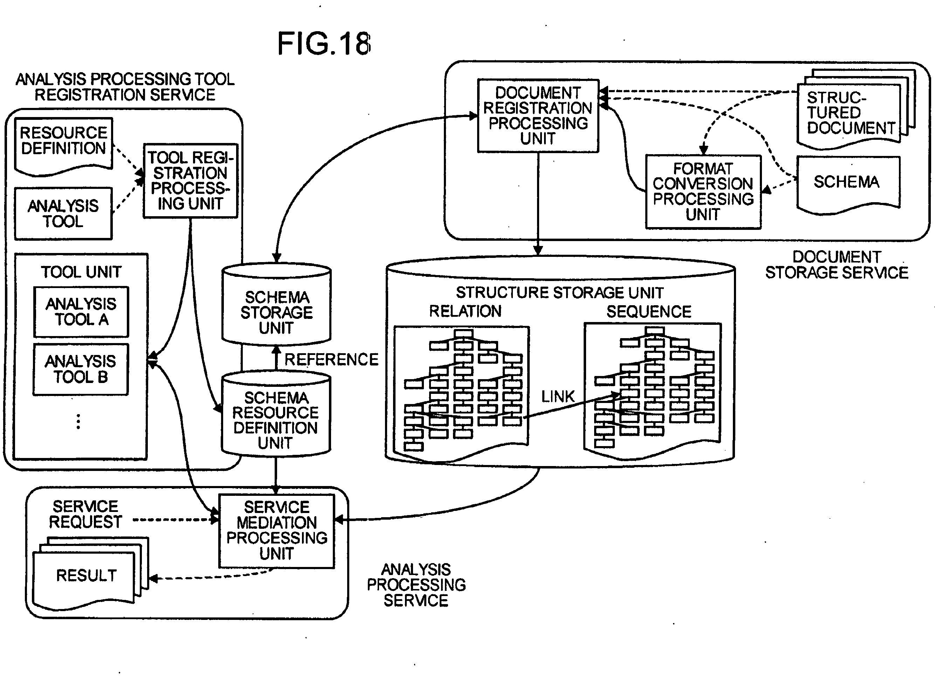

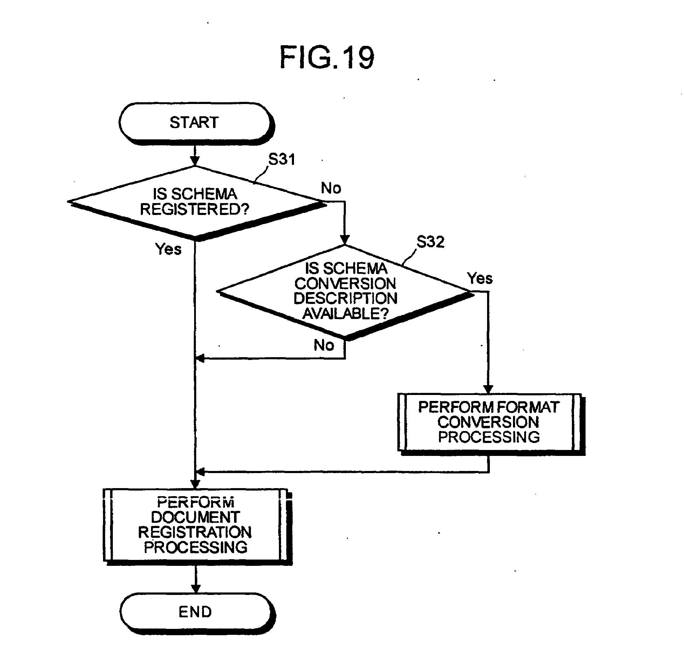

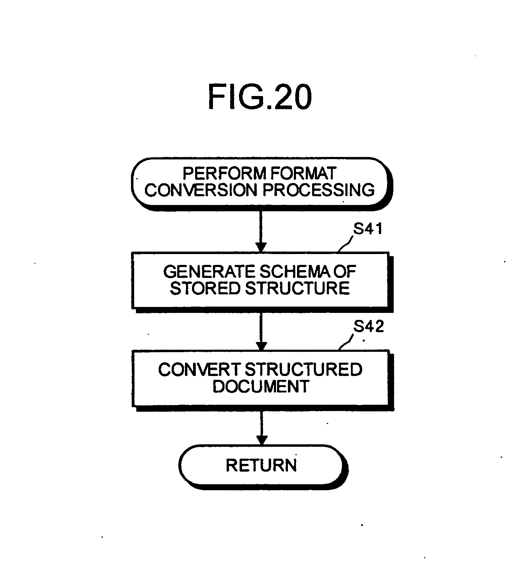

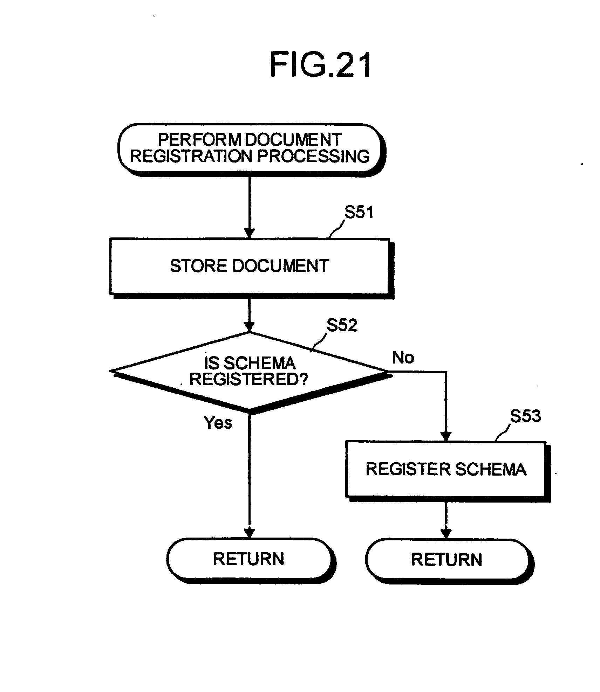

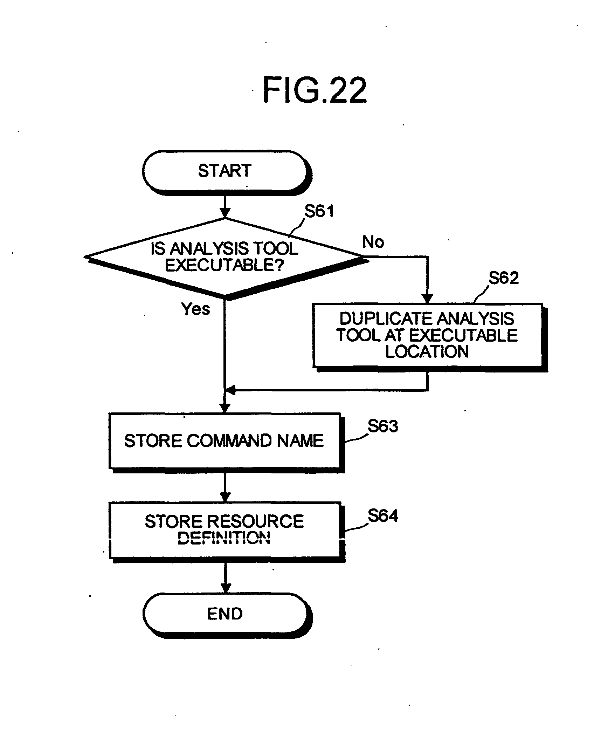

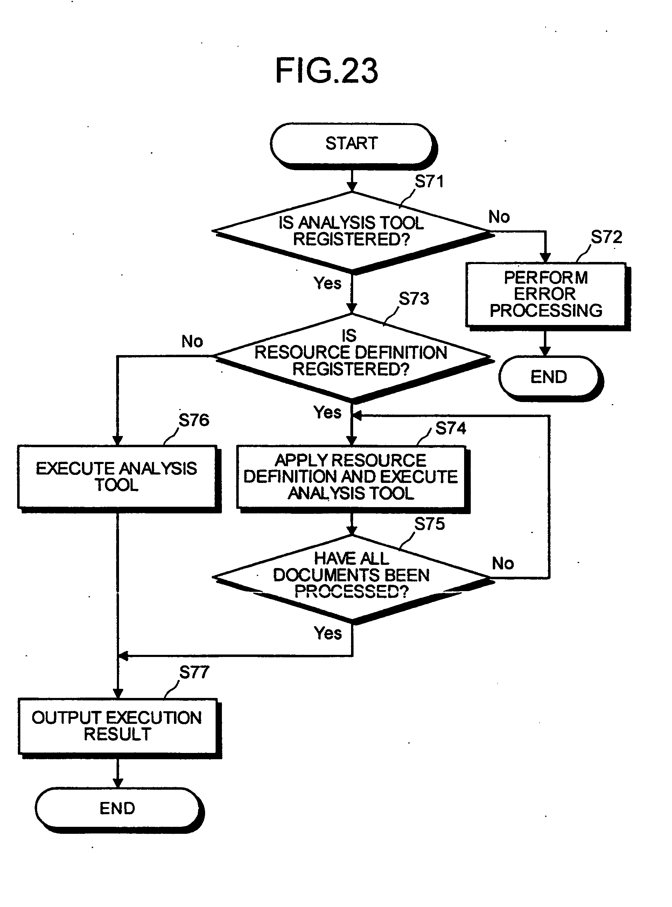

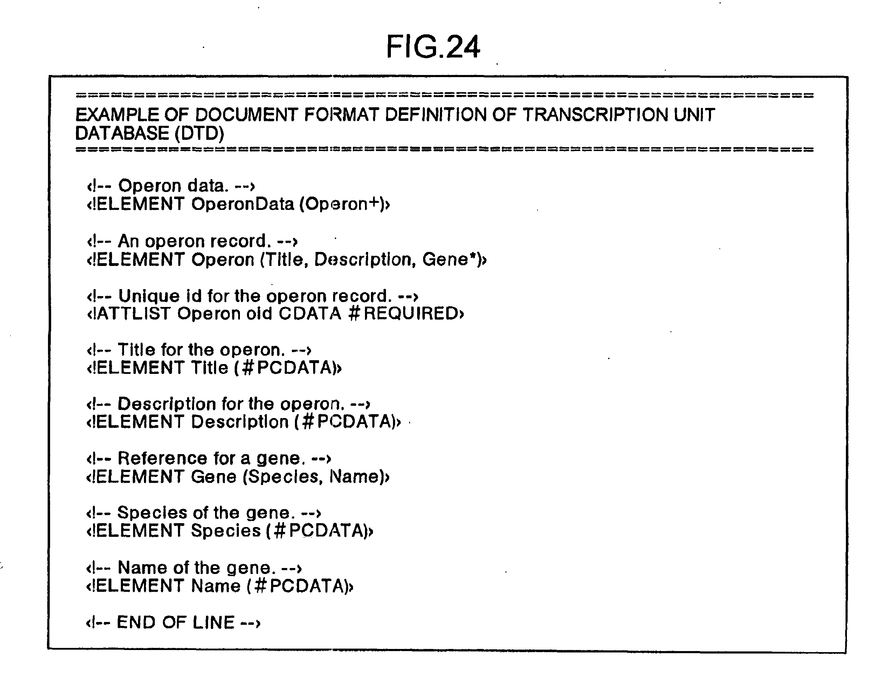

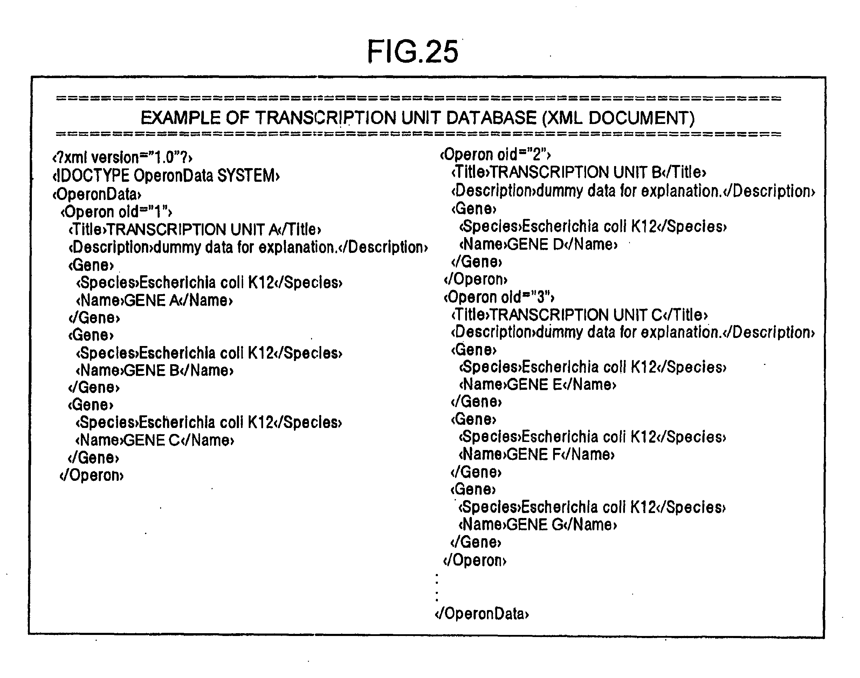

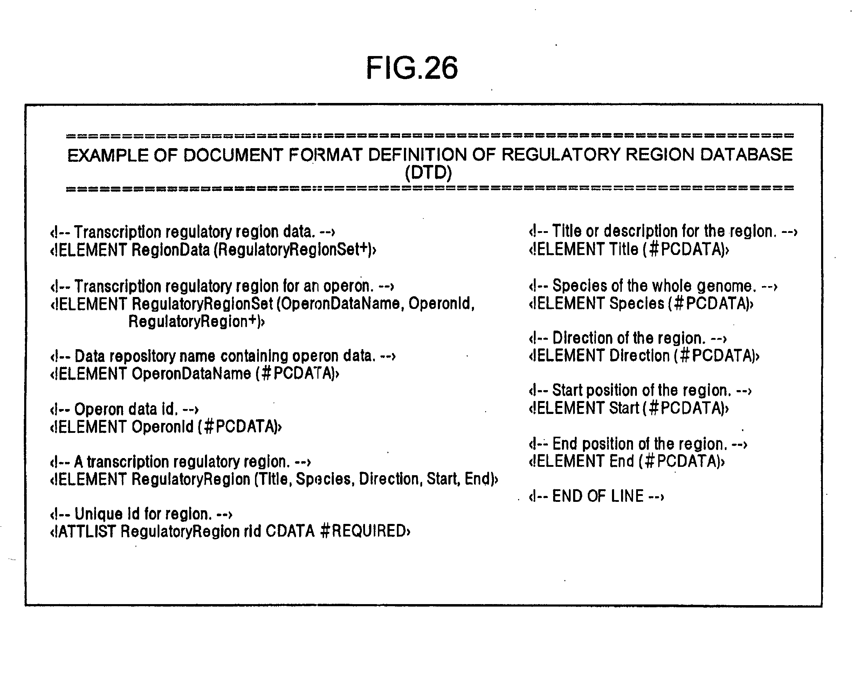

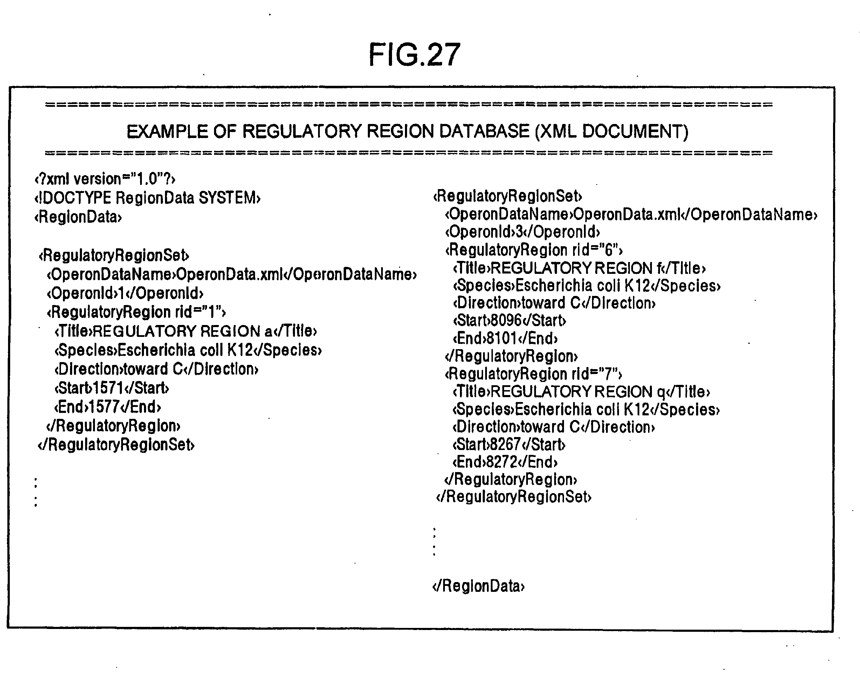

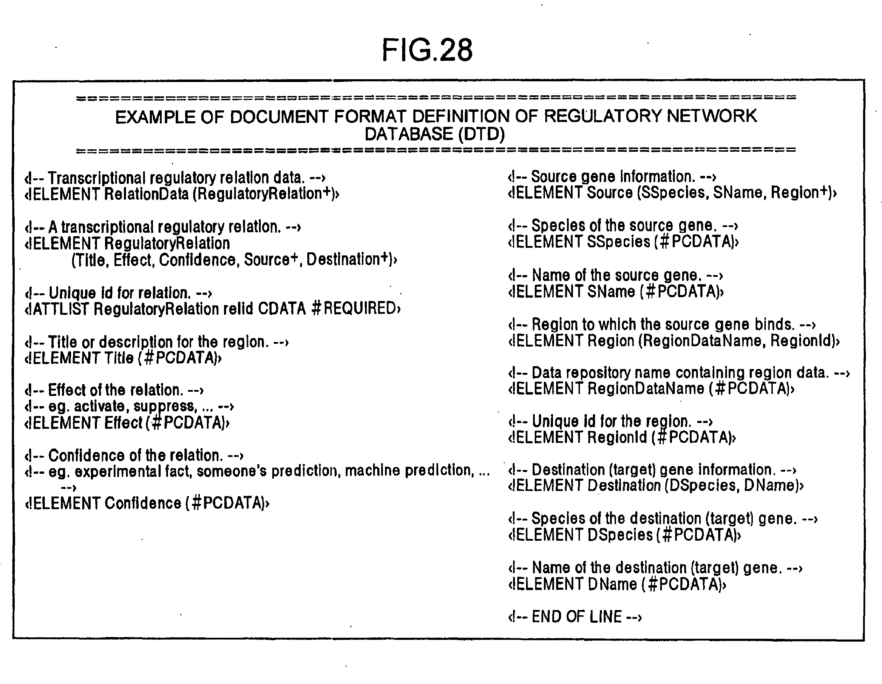

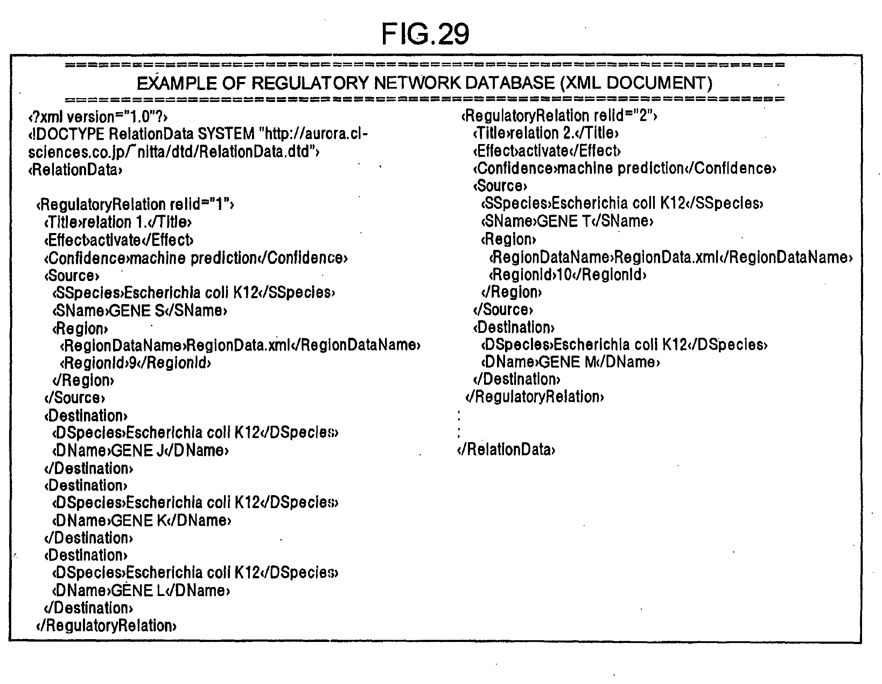

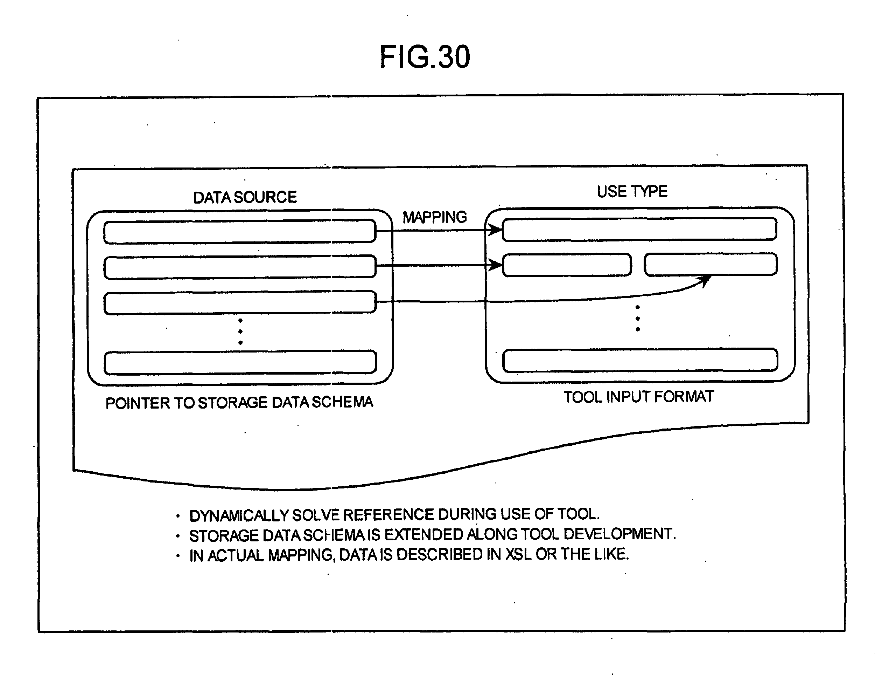

Fig. 1 is an illustration of one example of the basic data structure of a sequence information database of base sequences of genes or amino acid sequences of proteins; Fig. 2 is a block diagram illustrating one example of the configuration of a system to which the present invention is applied; Fig. 3 is a principle block diagram illustrating the basic principle of the present invention; Fig. 4 is a conceptual view of one example of the conversion of the format of acquired data according to the present invention; Fig. 5 is a flowchart of format conversion of an input data, performed by an analysis tool; Fig. 6 is an illustration of one example of schema format conversion instruction information on sequence information described in XSL; Fig. 7 is an illustration of one example of structured data (an XML document) the format of which has been converted according to the schema format conversion instruction information illustrated in Fig. 6; Fig. 8 is an illustration of one example of schema data (DTD) the format of which has been converted according to the schema format conversion instruction information illustrated in Fig. 6; Fig. 9 is an illustration of one example of schema format conversion instruction information on document information described in XML; Fig. 10 is an illustration of one example of structured data (an XML document) the format of which has been converted according to the schema format conversion instruction information illustrated in Fig. 9; Fig. 11 is an illustration of one example of schema data (DTD) the format of which has been converted according to the schema format conversion instruction information illustrated in Fig. 9; Fig. 12 is a flowchart of the outline of a gene expression control analysis process; Fig. 13 is a conceptual view illustrating the outline of transcription unit prediction; Fig. 14 is a conceptual view illustrating the outline of regulatory region prediction; Fig. 15 is a conceptual view illustrating the outline of regulatory gene prediction; Fig. 16 is an illustration of the structural difference between data described in BSML and data described in BioML; Fig. 17 is an illustration of the concept of a structured data processing apparatus to which the present invention is applied; Fig. 18 is an illustration of the basic configuration of the structured data processing apparatus to which the present invention is applied; Fig. 19 is a flowchart of the main routine of a document storage service; Fig. 20 is a flowchart of the subroutine "format conversion processing" of the document storage service; Fig. 21 is a flowchart of the subroutine "document registration processing" of the document storage service; Fig. 22 is a flowchart of the processing of an analysis processing tool registration service; Fig. 23 is a flowchart of the processing of an analysis processing service; Fig. 24 is an illustration of one example in which the document format of the transcription unit database illustrated in Fig. 13 is described using DTD; Fig. 25 is an illustration of one example in which structured data in the transcription unit database illustrated in Fig. 13 is described using an XML document; Fig. 26 is an illustration of one example in which the document format in the regulatory region database illustrated in Fig. 14 is described using DTD; Fig. 27 is an illustration of one example in which structured data in the regulatory region database is described using an XML document; Fig. 28 is an illustration of one example in which the document format in the regulatory network database illustrated in Fig. 15 is described using DTD; Fig. 29 is an illustration of one example in which structured data in the regulatory network database illustrated in Fig. 15 is described using an XML document; and Fig. 30 is an illustration of the concept of schema resource definition information.

### BEST MODE FOR CARRYING OUT THE INVENTION

Exemplary embodiments of the present invention will be explained hereinafter in detail with reference to the accompanying drawings. This invention is not limited by these embodiments.

In the following embodiments, the present invention will be explained in relation to examples in which the present invention is applied to XML-based structured description languages and schema languages. However, the present invention is not limited to the examples and is similarly applicable to systems applicable to all other structured description languages and schema languages.

### [Outline of the Present Invention]

The outline of the present invention will be explained first, and the configuration and processing of the present invention will be explained thereafter in detail. Fig. 3 is a principle block diagram illustrating the basic principle of the present invention.

Generally, the present invention has the following basic features. First, structured data that is described in a structured description language and schema data that defines the structure of the structured data, are acquired from an external database through the Internet (step SA-1).

Examples of the well-known external database include sequence databases such as GenBank, European Molecular Biology Laboratory (EMBL), and DNA Data Bank of Japan (DDBJ), databases related to human genome map data such as Genome Data Base (GDB) and online mendelian inheritance in man (OMIM), amino acid sequence databases such as Protein Identification Resource (PIR), SWISS-PROT, and PRF, protein function databases such as PROSITE and BLOCKS, protein three-dimensional structure database such as Protein Data Bank (PDB), integrated databases such as Entrez, and document databases such as PubMed. Each of these databases is a collection of structured data that is described in a predefined structured description language, and schema data that is described in a predefined schema language and that corresponds to the structured data.

The structured description language for describing the structured data acquired from the external database may be one of XML, SGML, BioML, BSML, ASN.1, GAME, structured description languages extended therefrom, and structured description languages having equivalent description abilities thereto. The schema data may be described in one of DTD, XML schema, RELAX, schema languages extended therefrom, and schema languages having equivalent description abilities thereto.

Then, the acquired structured data and schema data is converted based on schema format conversion instruction information (step SA-2). Fig. 4 is a conceptual view of one example of conversion of the format of the acquired data.

As illustrated in Fig. 4, after the structured data described in the structured description language and the schema data described in the schema language are acquired from the external database, the acquired data is converted based on the predefined schema format conversion instruction information.

The schema format conversion instruction information may be data described in XSL, an extended language from XSL, or a tree structure conversion language having equivalent description ability thereto. If so, a conversion processing may be executed using a well-known XSLT (XSL Transformation) processor such as Xalan (APACHE XML PROJECT) or XT (James Clark).

Fig. 6 is an illustration of one example of the schema format conversion instruction information on sequence information described in XSL. Based on the schema format conversion instruction information, the format of the acquired structured data is converted into an XML document (illustrated in Fig. 7), and that of the acquired schema data is converted into the DTD format (illustrated in Fig. 8).

In the example of the converted DTD illustrated in Fig. 8, elements (referred to as ELEMENT) used in the structured data are Sequence, Title, Nucleotide, Peptide, Reference, RefTitle, and Id. These elements define the types of the respective elements. Among them, "Sequence" means base sequence data. "Sequence" in turn includes, as child elements, "Title" that means the description of the sequence in a natural language, "Nucleotide" that means a base sequence, "Peptide" that means an amino acid sequence converted from the base sequence, and "Reference" that means a reference document. "Reference" includes, as child elements, "RefTitle" that means the title of the reference document, and "Id" that means the reference number of the reference document.

Fig. 9 is an illustration of one example of the schema format conversion instruction information on document information described in XSL. Based on the schema format conversion instruction information, the format of the acquired structured data is converted into an XML document (illustrated in Fig. 10), and that of the schema data is converted into the DTD format (illustrated in Fig. 11).

In the example of the converted DTD illustrated in Fig. 11, elements (referred to as ELEMENT) used in the structured data are Literature, Title, Abstract, Link, and Id. These elements define the types of the respective elements. Among them, "Literature" means entire document data. "Literature" includes, as child elements, "Title" that means the title of the document, "Abstract" that means the abstract of the document, and "Link" that means a set of numbers as references to relevant sequence data. "Link" includes, as a child element, "Id" that means each reference number.

The schema format conversion instruction information has made it possible to convert the format of the acquired data described in a different structured language or a different schema language into a predefined format or a format set at need. It is, therefore, possible to maintain consistency in the data acquired from various external databases, and ensure high extensibility for the data description form. This further enables access to the external databases that support the various data description formats. That is, it is possible to manage the internal database using a uniform, specific structured description language (for example, BSML or BioML), and thereby greatly improve database utilization efficiency.

Further, even if a new resource (for example, an XML element) is added to the schema, the format of the schema data can be easily converted into the newly added form.

In addition, the present invention is not limited to the example of acquiring data from external databases. Even if data is acquired from an internal database managed by the processing apparatus, the format of the internal data can be similarly converted by batch processing.

Referring back to Fig. 3, the present invention registers the converted structured data and schema data in respective databases (step SA-3).

The databases used may be any of the well-known XML storage systems (for example, a DOM (Document Object Model) tree storage system such as eXcelon or Tamino, an XML native storage system, an RDB wrapper storage system or one of processing systems having equivalent functions thereto).

The various databases, in which the converted data is registered at step SA-3, are accessed. A tool program (hereinafter, "an analysis tool") for data processing and a schema resource definition information for defining the schema resources of the structured data to be used to run the tool program, are registered in a corresponding manner (step SA-4).

The concept of the schema resource definition information is explained next, with reference to Fig. 30. The schema resource definition information may be information that defines the correspondence of registered data sources to the respective resources in a format for the use of the tool. For example, the schema resource definition information may be a mapping between the schema data of the structured data registered in the database and the input format of various tools. In addition, the schema resource definition information may be data described in XSL, an XSL extended language, or a tree structure conversion language having a description ability equivalent to that of XSL or the XSL extended language.

In Fig. 3, when the analysis tool is started (step SA5), the structured data and the schema data registered in the databases are dynamically converted based on the schema resource definition information (step SA-6), and the converted data is provided as input to the analysis tool (step SA-7).

An format conversion of the input data, performed by the analysis tool, is illustrated in Fig. 5. When a user starts a registered analysis tool A (step SB-1), the analysis tool A is read (loaded) from an analysis tool containing file (see Fig. 3) and a CPU converts the analysis tool A into an executable (step SB-2).

The schema resource definition information A (for example, an XSL document), corresponding to the analysis tool A, is acquired from the schema resource definition file (step SB-3).

The formats of the structured data and schema data registered in the respective databases are converted, based on the acquired schema resource definition information A (step SB-4).

The converted structured data and schema data are set as input data of the analysis tool A (step SB-5). The analysis tool thus completes the conversion.

The conversion processing at the step SA-6 in Fig. 3 may be executed using a well-known XSLT processor such as Xalan (APACHE XML PROJECT) or XT (James Clark).

The analysis tool processing results are registered in the respective databases, and are output to an output device (step SA-8).

The process for starting three types of analysis tools to execute a gene expression control analysis, and registering the processing results in the various databases is explained next, with reference to Figs. 12 to 15, and 24 to 29.

Fig. 12 is a flowchart of the outline of a gene expression control analysis process.

A transcription unit prediction tool to predict a transcription unit is started (step SC-1). Fig. 13 is a conceptual view illustrating the outline of the transcription unit prediction.

First the various external databases are accessed to acquire various pieces of data. The data formats are converted as needed to create a common database in advance.

The transcription unit prediction tool accesses the common database based on the corresponding schema resource definition information, processes as input data, the data that has been converted into an appropriate format, and registers processing results in a transcription unit database. The schema resource definition information of the transcription unit prediction tool is mapped onto the data input to the transcription unit prediction tool in the form of a set containing (gene name, start position, end position) for each gene from a gene name database. That is, the data on each gene registered in the gene name database is converted into the format of (gene name, start position, end position) based on the schema resource definition information of the transcription unit prediction tool to become input data to the transcription unit prediction tool. In the transcription unit database, the genes are grouped according to transcription units.

One example of the schema data and structured data stored in the transcription unit database is explained below with reference to Figs. 24 and 25.

Fig. 24 is an illustration of one example in which the document format of the transcription unit database is described using DTD. Fig. 25 is an illustration of one example in which the structured data in the transcription unit database is described using an XML document.

Referring back to Fig. 12, in the present invention, a regulatory region prediction tool is started and a regulatory region is predicted (step SC-2). Fig. 14 is a conceptual view illustrating the outline of the regulatory region prediction.

The started regulatory region prediction tool accesses the common database based on corresponding schema resource definition information to process various data. These data that are input to the regulatory region prediction tool include data in the appropriately converted form, data on the processing results of a sequence statistic processing tool such as BLAST (Basic Local Alignment Search Tool), and data registered in the transcription unit database. The regulatory region prediction tool also registers the processing results in the regulatory region database. The schema resource definition information of the regulatory region prediction tool is mapped onto the input data of the regulatory region prediction tool in the format of (transcription unit identifier, start position, end position, amino acid sequence of arbitrary length) for each transcription unit from the transcription unit database, the gene name database, and a whole genome database. In addition, the schema resource definition information of the regulatory region prediction tool is mapped onto the input data of the regulatory region prediction tool in the format of (amino acid partial sequence, number of expression in genome) for each of expressing combinations of amino acid distribution sequences having arbitrary lengths according to the processing results of the sequence statistic processing tool. Further, the schema resource definition information of the sequence statistic processing tool such as BLAST is mapped on the input data of the sequence statistic processing tool so as to fetch entire sequences from the whole genome database. The regions of sequence predicted to extend transcription unit record, are stored to regulate transcription.

Examples of the schema data and structured data stored in the regulatory region database will now be explained with reference to Figs. 26 and 27.

Fig. 26 is an illustration of one example in which the document format of the schema data in the regulatory region database is described in DTD. Fig. 27 is an illustration of one example in which the structured data in the regulatory region database is described using an XML document.

Referring back to Fig. 12, in the present invention, a regulatory gene prediction tool is started, and a regulatory gene is predicted (step SC-3). Fig. 15 is a conceptual view illustrating the outline of the regulatory gene prediction.

The started regulatory gene prediction tool accesses the common database based on corresponding schema resource definition information to process various data. These data that are input to the regulatory gene prediction tool include data in appropriately converted form, data on the processing results of the sequence statistic processing tool such as BLAST, data registered in the transcription unit database. The regulatory gene prediction tool also registers the processing results in a regulatory network database. The schema resource definition information of the regulatory gene prediction tool is mapped onto the input data of the regulatory gene prediction tool in the format of (gene name, amino acid sequence) for each of genes of DNA binding proteins from the sequence database. In addition, the schema resource definition information of the regulatory gene prediction tool is mapped onto the input data of the regulatory gene prediction tool in the format of (transcription unit identifier, list of regulatory region (start position, end position, amino acid sequence)) for each transcription unit from the transcription unit database and the whole genome database.

Examples of the schema data and structured data stored in the regulatory network database will be described with reference to Figs. 28 and 29.

Fig. 28 is an illustration of one example in which the document format of the schema data in the regulatory network database is described using DTD. Fig. 29 is an illustration of one example in which the structured data in the regulatory network database is described using an XML document.

At this point, the gene expression regulatory analysis processing illustrated in Fig. 12 is finished.

As can be seen, even if an item is added as needed by each analysis tool and the added item is used in a processing by a later analysis tool processing, it is possible to easily ensure the extensibility of each data to be used without changing the specification of the analysis tool. It is also possible to convert the format of the common database by batch processing.

### [System Configuration]

The configuration of the system according to the present invention will be explained next. Fig. 2 is a block diagram illustrating one example of the configuration of the system to which the present invention is applied. Among all the components of the system, only the components related to the present invention are conceptually illustrated. This system is generally constituted so that a structured data processing apparatus 100 and an external system 200 are connected to communicate with each other through a network 300.

The network 300 may be, for example, a network such as the Internet.

The external system 200 provides to a user, external databases related to sequence information and websites for executing external programs such as a homology search program and a motif search program.

The external system 200 may consist of a server such as a WEB server, or an ASP server, and the hardware of the external system 200 may consist of a commercially available information processing apparatus such as a workstation or a personal computer and attachments thereto. The respective functions of the external system 200 are realized by a CPU, a disk device, a memory device, an input device, an output device, a communication controller and the like in the hardware configuration of the external system 200 as well as programs controlling these components.

The structured data processing apparatus 100 generally consists of a control unit 102, such as a CPU, which controls the entire structured data processing apparatus 100 in the block, a communication control interface unit 104 connected to a communication equipment (not shown), such as router, connected to a communication line or the like, an input/output control interface unit 108 connected to the input device 112 and the output device 114, and a storage unit 106 which stores various databases and tables. These components are interconnected through an arbitrary communication path. Further, the structured data processing apparatus 100 is connected to the network 300 to be communicable therewith through a communication equipment such as a router and a wired or radio communication line such as a dedicated line.

Various databases and tables (a database for storing structured data 106a to a processing result database 106f) included in the storage unit 106 are storage units in a fixed disk device. These components store various programs, tables, files, databases, webpage files, used in various processings.

Among the components of the storage unit 106, the database for storing structured data 106a stores structured data.

The database for storing schema data 106b stores schema data.

The schema format conversion instruction information file 106c stores schema format conversion instruction information.

The analysis tool containing file 106d stores information and the like related to analysis tools.

The schema resource definition file 106e stores schema resource definition information and the like.

The processing result database 106f stores information related to processing results of the analysis tool.

The communication control interface unit 104 controls communication between the structured data processing apparatus 100 and the network 300 (or communication equipment such as a router). That is, the communication control interface unit 104 functions to communicate data to other terminals through the communication line.

The input/output control interface unit 108 controls the input device 112 and the output device 114. An output device such as a monitor (including a home television) or a speaker may be used as the output device 114 (note, the output device will be sometimes denoted as "monitor" hereinafter). An input device such as a keyboard, a mouse, or a microphone can be used as the input device 112. The monitor realizes a pointing device function in cooperation with the mouse.

The control unit 102 includes an internal memory for storing, for example, a control program of an OS (Operating System), a program that specifies various processing procedures, and required data. The control unit 102 performs information processing to execute various processings using these programs. Conceptually, the control unit 102 consists of a structured data acquisition unit 102a, a format conversion unit 102b, a structured data registration unit 102c, an analysis tool registration unit 102d, an analysis tool start unit 102e, and a processing result registration unit 102f.

The structured data acquisition unit 102a acquires structured data that is described in a structured description language and schema data that defines the structure of the structured data.

The format conversion unit 102b converts the formats of the structured data and schema data acquired by the structured data acquisition unit 102a based on the schema format conversion instruction information.

The structured data registration unit 102c registers in databases, the structured data and schema data converted by the format conversion unit 102b.

The analysis tool registration unit 102d registers a tool program for accessing the databases in which the structured data and the schema data are registered to perform data processing, and schema resource definition information that defines the schema resources of the structured data input to the tool program, so that the tool program and the schema resource definition information correspond to each other.

The analysis tool start unit 102e dynamically converts the structured data and schema data registered in the databases based on the schema resource definition information, and inputs the converted structured data and schema data to the tool program when the tool program is started.

The processing result registration unit 102f registers the processing results of the analysis tool in the databases.

The detail of processings performed by these components will be explained later in detail.

### Examples

Exemplary processing of the system constituted as explained above in this embodiment will next be explained in detail with reference to Figs. 17 to 23.

Fig. 17 is an illustration of the concept of the structured data processing apparatus to which the present invention is applied.

The structured data processing apparatus according to the present invention includes databases illustrated in Fig. 17. The databases contain a plurality of sub-databases. The sub-database "sequence database" stores sequence data. Although only one sequence database is shown, the processing apparatus may include a plurality of sequence databases.

Each record of the sequence database includes at least a base or amino acid sequence data body. The record may include partial modification description and whole description in BSML, BioML or GAME.

Data related to multiple pieces of sequence data is stored in sub-databases labeled "relational database" separate from the sequence database. Fig. 17 illustrates four relational databases A to D.

Each record of each relational database includes at least one reference information. The reference information indicates the entire records of the sub-databases in the system or external databases, or a specific part in the record. Each record may include fields such as partial modification description, and whole description. Arrows labeled "reference" indicate that the relational database "D", for example, includes one or more record having references to the sequence database and the other relational databases "A" to "C".

Fig. 18 is an illustration of the basic configuration of the structured data processing apparatus, that is, a database system, to which the present invention is applied. This system consists of a basic processing module, an extension processing module, and a storage unit.

The basic processing module consists of a tool registration processing unit (conceptually corresponding to the analysis tool registration unit 102d in Fig. 2), a document registration processing unit (conceptually corresponding to the structured data registration unit 102c in Fig. 2), a format conversion processing unit (conceptually corresponding to the format conversion unit 102b in Fig. 2), a service mediation processing unit (conceptually corresponding to the analysis tool start unit 102e and the processing result registration unit 102f in Fig. 2), and a link processing unit. The extension processing module consists of a plurality of tool units (analysis tools A, B, ... in Fig. 18, which conceptually correspond to the analysis tool containing file 106d in Fig. 2). The storage unit consists of a structure storage unit (conceptually corresponding to the database for storing structured data 106a in Fig. 2), a schema storage unit (conceptually corresponding to the database for storing schema data 106b in Fig. 2), a schema resource definition unit (conceptually corresponding to the schema resource definition file database 106e in Fig. 2), and a result file (conceptually corresponding to the processing result database 106f in Fig. 2).

Broadly, the system in Fig. 18 provides three services. These are an analysis processing tool registration service provided by the tool registration processing unit, a document storage service provided by the document registration processing unit, and an analysis processing service (including a search processing service) provided by the service mediation processing unit.

In the analysis processing tool registration service, the tool registration processing unit reads an analysis tool and a corresponding resource definition, and registers the analysis tool in the tool unit and the resource definition in the schema resource definition unit.

In the document storage service, the document registration processing unit reads a structured document with its document format such as DTD, XML-Schema and RELAX clearly specified therein, conducts a format conversion processing of the document as needed, and stores the converted document in the structure storage unit. The document registration processing unit next inquires the schema storage unit if the document format of the structured document (one or many structured documents) is already registered. If the document format is already registered, the document registration processing unit does not do any processing. However, if the document format is not registered, the document registration processing unit acquires the document format and registers the document format in the schema storage unit.

In the analysis processing service, the service mediation processing unit receives a service request, and determines which analysis processing tool is necessary to execute the requested service. The service mediation processing unit acquires the resource definition corresponding to the analysis processing tool from the schema resource definition unit. The service mediation processing unit acquires a set of documents from the structure storage unit, while resolving links with document data needed for execution. The service mediation processing unit also requests the analysis processing tool to process the set of document data to generate processing results.

Each thick arrow in Fig. 18 signifies the movement of data. However, the arrows from the structure storage unit do not always signify the actual data movement but often signify the movement of only reference information (a pointer).

In one aspect of the present invention, the structured data processing apparatus according to the present invention manages information related to base sequences of genes or amino acid sequences of proteins. The processing includes a sequence data storage unit that stores sequence data related to the base sequences or the amino acid sequences, and many relational data storage units that store relational data related to the base sequences or the amino acid sequences. The information on the entire base sequences or amino acid sequences is stored in the sequence data storage unit or the relational data storage units. Each of relational data records stored in the relational data storage units includes a reference structure for reference to the relational data storage unit itself or a reference structure for reference to entirety or part of data records that constitute the sequence data storage unit.

Further, the structured data processing apparatus according to the present invention includes a basic processing unit, an extension processing unit, and a storage unit. The basic processing unit preferably includes a tool registration unit that reads an analysis tool and a resource definition paired with the analysis tool, and registers the analysis tool and the resource definition; a document registration unit that reads structured data with its document format specified therein, conducts a format conversion processing of the structured data at need, and registers the structured document in the storage unit; a service mediation unit that receives a service request, and determines an analysis processing tool necessary to execute a requested service; and a link processing unit that refers to the reference structure. The extension processing unit preferably includes many types of analysis processing tools executing an analysis processing of the structured document. The storage unit preferably includes a structure storage unit that stores the structured document read by the document registration unit; a schema storage unit that stores a schema of the structured document; and a schema resource definition unit that stores the resource definition registered by the tool registration unit. The structure storage unit stores the structured document while maintaining a tree structure of the structured document.

It is also preferable that the structured data processing apparatus according to the present invention includes a conversion unit which reads data from an external database, and converts the data into data to be stored in the sequence data storage unit or the relational data storage units.

Further, it is preferable that the structured data processing apparatus according to the present invention includes a search unit that searches the sequence data storage unit or the relational data storage units, and outputs a search result as a structured document.

Moreover, it is preferable that in the structured data processing apparatus according to the present invention, the search unit converts a format of the structured data into a description format of BSML (Bio Sequence Markup Language).

Additionally, it is preferable that in the structured data processing apparatus according to the present invention, the search unit converts the format of the structured data into a description format of BioML (BIO polymer Markup Language).

The outline of the processings of the embodiment of the present invention will be explained hereinafter in detail with reference to the drawings. The structured data processing apparatus (system) is constituted as illustrated in Fig. 18. In this embodiment, a constitution method for attaining a specific object will be concretely explained. The specific object is a service for inputting a base sequence and searching other base sequences related to the base sequence. The related sequences are searched as follows.

A document record closer in natural language to the input base sequence is obtained first from document records linked to the record that includes the base sequence. Base sequences included in this record become search results. Such a method for searching related sequences using document data will be referred herein as "a literature similarity method". According to the literature similarity method, the number of hits can be controlled by increasing or decreasing the number of the records (two in the above explanation) of a document DB interposing between two sequences.

As explained, this system provides three services. In this embodiment, a plurality of services such as command services, library services, TCP/IP services, and http services (CGI) may be considered. For brevity, the system is assumed to provide the command services.

In an operative state, the system can execute the following service commands:
(1) A document storage service command;
(2) An analysis processing tool registration service command; and
(3) An analysis processing service command.

The service command (2) depends on the storage conditions of the service command (1), and the service command (3) depends on the storage and registration conditions of the service commands (1) and (2). The conditions will be explained later in detail.

### (1) Document Storage Service Command

The document storage service command (1) is executed as follows:
store <document name> <schema name> [<schema conversion description name>].

In the command (1), "store" is the name of the document storage service command. The file name of an XML document to be stored is specified by <document name>. The file name of the document format definition (DTD) of the XML document to be stored is specified by <schema name>. The name of a file that describes a conversion instruction for convertingthe schema of the XML document to be stored into a schema for this system in an XSL language, is specified by <schema conversion description name>. If the structured data is stored in the structure storage unit without converting the format of the data, the schema conversion description name may be omitted.

Figs. 19 to 21 are flowcharts of the processings of the document storage service.

Fig. 19 is a flowchart of the main routine of the document storage service and the steps therein are as explained below.

At step S31, it is determined whether the schema of the structured document to be stored is registered in the schema storage unit.

If it is determined at the step S31 that the schema is not registered in the schema storage unit ('NO' at step S31), it is determined whether schema conversion description is available (step S32). If it is determined at step S31 that the schema is registered in the schema storage unit ('YES' at step S31), the processing goes to a subroutine to perform document registration processing. The subroutine for document registration processing is explained later with reference to Fig. 5.

If it is determined at step S32 that the schema conversion description is available ('YES' at step S32), the processing goes to a subroutine to perform format conversion processing. The subroutine for format conversion processing will be explained later with reference to Fig. 20. If it is determined at step S32 that the schema conversion description is unavailable ('NO' at step S32), the processing goes to the subroutine for document registration processing.

Fig. 20 is a flowchart of the subroutine "format conversion processing" in the document storage service.

At step S41, the schema of the storage structure is generated from the schema of the structured document to be stored and the schema conversion description.

At step S42, the structured document is converted according to the schema conversion description, and the conversion result and the schema generated at step S41 are passed to the subroutine for document registration processing. Ordinarily available XSLT processor (Saxon, Xalan, or the like) or a processing system equivalent in function to the XSLT processor is used for the conversion.

Fig. 21 is a flowchart illustrating the subroutine "document registration processing" in the document storage service.

At step S51, the document is stored in the structure storage unit. A commercially available XML storage system (DOM tree storage such as eXcelon or Tamino, an XML native storage, an RDB wrapper storage, or a processing system equivalent in function thereto) is used as the storage.

At step S52, it is determined whether the schema is registered in the schema storage unit.

If it is determined at step S52 that the schema is not registered ('NO' at step S52), the schema is registered at step S53 and the processing is finished. If it is determined at step S52 that the schema is registered ('YES' at step S52), the processing is finished.

An example of executing the document storage service is explained below.

In this execution example, the document is expressed in XML, and the schema is expressed in XML DTD. The data to be stored is expressed as an XML document using the following URL service. The sequence data is obtained from the GenBank service, and the document data is obtained from the PubMed service (see http://www.ncbi.nlm.nih.gov/Genbank/). References to the data and schemas that can be directly acquired from the GenBank are not illustrated.

It is assumed that the schema conversion description of the sequence data is 'sequence.xsl' (Fig. 6) and that the schema conversion description of the document data is 'literature.xsl' (Fig. 9). These data are input to the format conversion processing.

After the format conversion processing, the data subjected to the document registration processing is converted as follows.

The description of the sequence data is converted into 'sequence.xml' (Fig. 7) and the description of the schema data is converted into 'squence.dtd' (Fig. 8).

'Sequence' tag means an entire sequence, 'Title' tag means an explanation related to the sequence in a natural language, 'Nucleotide' tag means a base sequence, 'Peptide' tag means an amino acid sequence converted from the base sequence, 'Reference' tag means a reference document, 'RefTitle' tag means the title of the reference document, and 'Id' tag means the reference number of the reference document.

Further, the description of one record of the document data is converted into 'literature.xml' (Fig. 10) and that of the schema is converted into 'literature.dtd' (Fig. 11).

'Literature' tag means the entire document data, 'Title' tag means the title of the document, 'Abstract' tag means the abstract of the document, 'Link' tag means a set of numbers as references to related sequence data, 'Id' tag means an individual reference number.

### (2) Analysis processing tool registration service command

The analysis processing tool registration service command (2) is executed as follows:
register <tool command name> [<resource definition>].

In the command (2), "register" is the name of the analysis processing tool registration service command. The name of a file that describes a conversion instruction for converting the format of the data schema for the storage of this system into a data format used to input the tool in an XSL language is specified in <tool command name>. If the data input to the tool is not the data contained in the storage unit, the resource definition may be omitted.

Fig. 22 is a flowchart of the processing of the analysis tool registration service that is started in response to the register command, and is executed according to the following steps.

At step S61, it is determined whether the analysis tool is executable.

If it is determined at step S61 that the analysis tool is not executable ('NO' at step S61), the analysis tool is duplicated at a location where this system can execute the tool (step S62).

If it is determined at step S61 that the analysis tool is executable ('YES' at step S61) or after the analysis tool is duplicated at step S62, the command name of the analysis tool is stored at step S63.

At step S64, the resource definition is stored in the schema resource definition unit and the processing ends.

An example of executing the analysis tool registration service will be explained.

In this example of execution, two analysis processing tools (for an indexing processing and a search processing) for conducting a sequential search on the sequence data and the document data stored in the system using the literature similarity method are registered according to the above steps.

In the indexing processing, 1 h-index command is used. In the search processing, 1h-search command is used. The 1h-index command uses, as a factor, all search target data consisting of a set of pairs of search target character strings and identifiers. This command is registered together with the resource definition 1h-index.xsl. The 1 h-search command uses, as a factor, a search key or sequence. No resource definition is registered together with this command.

### (3) Analysis processing service command

The analysis processing service command (3) is executed as follows:
process <analysis tool name> [-toolargs<tool factor list>] [-serviceargs<service factor list>]

In the command (3), "process" is the name of the analysis processing service command. The name of the analysis tool already registered in the system is specified in <analysis tool name>. A parameter passed to the analysis tool is specified in <tool factor list>. If the analysis tool does not need an additional factor, the tool factor list may be omitted. A parameter that is not directly passed to the analysis tool but that is necessary for the service is specified in <service factor list>. If an additional factor is not necessary, the service factor list may be omitted.

Fig. 23 is a flowchart of the processing of the analysis processing service that is started in response to the process command and is executed by the service mediation processing unit according to the following steps.

At step S71, the service mediation unit determines whether an analysis tool is registered in the system.

If the service mediation unit determines that the analysis tool is not registered ('NO' at step S71), the service mediation unit performs an error processing at step S72.

If the service mediation unit determines that the analysis tool is registered ('YES' at step S71), the service mediation unit determines whether a resource definition corresponding to the analysis tool is registered in the schema resource definition unit.

If the service mediation unit determines that the corresponding schema definition is registered ('YES' at step S73), the service mediation unit applies the resource definition (XSL) to each document in the structure storage unit (using the service factor list, if any), and applies the analysis tool to each result. At step S75, the service mediation unit determines whether all the documents have been processed. Thus, the service mediation unit repeatedly executes step S74 until all the documents are processed ('YES' at step S75).

If the service mediation unit determines that the resource definition is not registered ('NO' at step S73), the service mediation unit executes the analysis tool (step S76).

After executing the analysis tool at step S76 or after finishing the processing at step S75, the service mediation unit outputs an execution result and the processing ends.

An example of executing the analysis processing tool registration service is explained next.

As already explained, the literature similarity method is mounted in the system by the two analysis tools, i.e., the 1 h-index command for the indexing processing and the 1 h-search command for the search processing.

In the indexing processing, the process command is started as follows:
process 1 h-index -toolargs "@documents" -serviceargs" -depth=2"

In the 1 h-index tool, 1 h-search.xsl is present as the resource definition. Therefore, the 1 h-index tool conducts an XSLT processing on all the documents stored in the structure storage unit. This processing uses the resource definition "1h-index.xsl" and the service factor "-depth=2" and performs the following.

A literature record set referred to by each respective sequence record 's' in the structure storage unit is assumed as "L1". A sequence record set referred to by each literature record 1 of L1 is assumed as "S1".

A literature record set referred to by each sequence record S' of S1 is assumed as "L2". In this way, only parts having natural language (text) data from all the sets obtained by tracing back a path of sequence-literature pairs by two pairs (the number of pairs is designated by "-depth=2"), are fetched together with Id of the original sequence 's'. This XSLT processing result is passed to 1h-index (the way of passing is defined by "-toolargs"@documents"") to create an index.

In the search processing, the process command is started as follows:
process 1h-search -toolargs "<sequence ID>..."

Since no resource definition is present in the 1 h-search tool, the 1h-search is directly started and a set of sequence ID's related to the sequence ID are output as a result using the index created by 1h-index tool.

As explained so far, according to the present invention, the relational DB can be extended independently of the sequence DB. This facilitates the extension of schemas which cannot be contained in the frameworks of the sequence DB records, thereby solving Problem (1).

Further, the present invention includes the document storage unit of a structure storage type and the relational DB for referring to the partial structures of the records. Therefore, it is possible to integrally, efficiently convert the formats of data into various formats that are different in structure, thereby solving Problem (2).

In addition, as explained in the embodiment of the invention with the example of the literature similarity method, this system realizes both flexibility and mounting efficiency, thus solving Problem (2). The performance of this system is more conspicuous when the structure storage unit is implemented by the native structure storage technique rather than the RDB technique.

In the example of mounting the literature similarity method, a processing target text part is dynamically created using XSLT during the creation of the index. This makes it possible to express the number of link stages by parameters and improve the flexibility of executable functions. As for the efficiency problem, in the mechanism of combining the analysis tools according to the command lines explained in the embodiment, data is passed in the form of byte streams. This may hamper the improvement of efficiency. However, this problem can be solved by using a component combining technique such as sharing a data space.

Furthermore, analysis components other than the literature similarity method component can be flexibly added by preparing an instruction to generate a document required by a tool from the document formats registered in the schema storage unit. Even if many structured document formats are registered, they can be stored temporarily in the structure storage unit, thus demonstrating the flexibility of the system.

### [Other Embodiments]

The embodiments of the present invention have been explained above. However, the present invention can be carried out by various embodiments other than the embodiments within the scope of the technical concept defined by the claims.

The example in which the structured data processing apparatus 100 performs processing in a standalone manner has been explained above. Alternatively, the structured data processing apparatus 100 may perform a processing in response to a request from a client terminal different in structure from that of the processing apparatus 100, and return the processing result to the client terminal.

Among the processings explained in the embodiments, all of or part of those explained to be automatically carried out can be carried out manually and all of or part of those explained to be manually carried out can be carried out automatically.

Further, the processing steps, control steps, concrete names shown in the documents or drawings, information including parameters such as various pieces of registration data and search conditions, example of screens, and database configurations can be arbitrarily changed unless specified otherwise.

Additionally, the respective components of the structured data processing apparatus 100 shown in the drawings are conceptually functional elements. Therefore, the processing apparatus 100 is not always physically constituted as shown in the drawings.

For example, all of or arbitrary part of the respective elements of the structured data processing apparatus 100 or the processing functions of the respective elements, particularly those carried out by the control unit 102 can be realized by a CPU (Central Processing Unit) and programs interpreted and executed by the CPU. Alternatively, they can be realized as wired logic hardware. The programs are recorded in a recording medium to be explained later and mechanically read by the structured data processing apparatus 100 as needed.

A computer program for issuing an instruction to the CPU, in association with an OS (Operating System), to make the CPU perform various processings is recorded in a storage unit 106 such as a ROM or HDD. This computer program is executed by being loaded onto memory such as a RAM, and the program as well as the CPU constitutes the control unit 102. Further, this computer program may be stored in an application program server connected to the structured data processing apparatus 100 through the arbitrary network 300, and may be downloaded either entirely or partially as needed.

The computer program according to the present invention can be stored in a computer readable recording medium. It is assumed herein that examples of the "recording medium" that temporarily stores the program include arbitrary "portable physical mediums" such as a flexible disk, a magneto-optical disk, a ROM, an EPROM, an EEPROM, a CD-ROM, an MO and a DVD, arbitrary "fixed physical mediums" such as a ROM, a RAM and a HD included in various types of computer systems, and "communication mediums", such as a communication line or a carrier wave used for transmitting the program through the network represented by a LAN, a WAN, or the Internet.

Further, "computer program" means a data processing method described in an arbitrary language or by an arbitrary description method, and may be of arbitrary type including a source code and a binary code. The "computer program" is not always limited to the program constituted unitarily. Examples of the program includes a program constituted to be distributed as a plurality of modules or libraries and a program which attains its function in association with a separate program represented by the OS (Operating System). Any well-known configuration and procedures can be used for implementing a concrete configuration to allow each processing apparatus shown in the embodiment to read the recording medium, the reading procedures, and the installation procedures after the reading.

The various databases (the database for storing structured data 106a to the processing result database 106f) stored in the storage unit 106 are storage units such as memory devices including a RAM and a ROM, fixed disk devices including a hard disk, a flexible disk, and an optical disk. These databases store various programs, tables, files, databases, and webpage files used to provide various processings and websites.

Further, the structured data processing apparatus 100 may be realized by installing thereon software (including a program, data, and the like) for connecting peripherals such as a printer, a monitor and an image scanner to an information processing apparatus such as a well-known personal computer or a workstation and allowing the information processing apparatus to realize the method of the present invention.

The concrete manners of distribution or integration of the structured data processing apparatus 100 are not limited to those shown in the drawings. The structured data processing apparatus 100 can be constituted to be either entirely or partially physically distributed or integrated in arbitrary unit according to the load. For example, the databases can be constituted independently as database units or part of the processings may be realized using the CGI (Common Gateway Interface).

Moreover, the network 300 may have a function of mutually connecting the structured data processing apparatus 100 and the external system 200. The network 300 may include any one of the Internet, intranets, LANs (including wired LAN and wireless LAN), a VAN, a personal computer communication network, public telephone networks (both analog and digital), dedicated line networks (both analog and digital), a CATV network, portable line exchange networks/portable packet exchange networks of IMT2000 type, GSM type, PDC/PDC-P type and the like, a wireless call network, a local wireless network such as Bluetooth, a PHS network, satellite networks such as CS, BS, and SDB and the like. That is, in this system, transmitting and receiving of various data can be made via either cable or wireless arbitrary network.

According to one aspect of the present invention, structured data described in a structured description language and schema data defining a structure of the structured data are acquired, the structured data and the schema data thus acquired are converted based on schema format conversion instruction information, the structured data and the schema data thus converted are registered in a database, a tool program for accessing the database, in which the structured data and the schema data are registered by the structured data registration unit, to conduct a data processing, and schema resource definition information which defines resources of a schema of the structured data input to the tool program are registered so that the tool program corresponds to the schema resource definition information, and the structured data and the schema data registered in the database are dynamically converted according to the schema resource definition information corresponding to the tool program and the converted structured data and schema data are input to the tool program if the tool program is started. Therefore, it is possible to provide a structured data processing apparatus, a structured data processing method, a program, and a recording medium capable of converting the formats of the acquired structured data and schema data described in different structured languages and schema languages into predetermined formats or formats as per the need.

According to another aspect of the present invention, it is possible to facilitate matching the data acquired from various external databases and ensure high extensibility related to data description formats. This consequently can facilitate accessing external databases that support various data description formats. That is, it is possible to manage an internal database in the format of the uniform, specific structured description language (for example, BSML or BioML). Therefore, it is possible to provide a structured data processing apparatus, a structured data processing method, a program, and a recording medium capable of greatly improving database utilization efficiency.

According to still another aspect of the present invention, even if a new resource (for example, an XML element) is added to the schema, it is possible to provide a structured data processing apparatus, a structured data processing method, a program, and a recording medium capable of easily converting the format of the schema into the newly added format.

According to still another aspect of the present invention, it is possible to provide a structured data processing apparatus, a structured data processing method, a program, and a recording medium capable of easily ensure the extensibility of the data to be used can be easily ensured without changing the specification of the analysis tool even if an item is added at need by each analysis tool and the added item is used in a processing by a later analysis tool processing.

According to still another aspect of the present invention, it is possible to provide a structured data processing apparatus, a structured data processing method, a program, and a recording medium capable of converting the format of a common database by batch processing.

According to still another aspect of the present invention, the structured description language is one of XML, SGML, BioML, BSML, ASN.1, GAME, structured description languages extended from these six languages, and structured description languages equivalent in description ability to these six languages. Therefore, it is possible to provide a structured data processing apparatus, a structured data processing method, a program, and a recording medium capable of efficiently converting the structured data, normally used in the bioinformatics field, described in these structured description languages.

According to still another aspect of the present invention, the schema data is data described in one of DTD, XML schema, RELAX, schema languages extended from these three languages, and schema languages equivalent in description ability to these three languages. Therefore, it is possible to provide a structured data processing apparatus, a structured data processing method, a program, and a recording medium capable of efficiently converting the schema data, normally used in the bioinformatics field, described in these schema languages.

According to still another aspect of the present invention, the schema format conversion instruction information and the schema resource definition information are data described in one of XSL, the language extended from the XSL, and tree structure conversion languages equal in description ability to the XSL and the XSL extended language. Therefore, it is possible to provide a structured data processing apparatus, a structured data processing method, a program, and a recording medium capable of efficiently converting the structured data and the schema data, normally used in the bioinformatics field, based on the schema format conversion instruction information and the schema resource definition information described in these schema conversion description languages.

According to still another aspect of the present invention, the structured data includes an element about at least one of sequence information, which includes one of or both of base sequences and amino acid sequences, and literature information. Therefore, it is possible to provide a structured data processing apparatus, a structured data processing method, a program, and a recording medium capable of acquiring sequence information registered in databases like GenBank or literature information registered in databases like PubMed, and converting the format of the acquired information.

### INDUSTRIAL APPLICABILITY

The structured data processing apparatus, the structured data processing method, the program, and the recording medium according to the present invention are suited for efficiency processing structured data in various formats defined by schema languages in various formats.

## Claims

1. A structured data processing apparatus comprising:
a structure data acquisition unit which acquires structured data described in a structured description language, and schema data defining a structure of the structured data;
a format conversion unit which converts the structured data and the schema data acquired by the structured data acquisition unit based on schema format conversion instruction information;
a structured data registration unit which registers the structured data and the schema data converted by the format conversion unit in a database;
an analysis tool registration unit which registers a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and
an analysis tool start unit which converts the structured data and the schema data registered in the database according to the schema resource definition information corresponding to the tool program, and which inputs the converted structured data and schema data to the tool program if the tool program is started.

2. The structured data processing apparatus according to claim 1, wherein
the structured description language is one of XML, SGML, BioML, BSML, ASN.1, GAME, structured description languages extended from these six languages, and structured description languages equivalent in description ability to these six languages.

3. The structured data processing apparatus according to claim 1 or 2, wherein
the schema data is data described in one of DTD, XML schema, RELAX, schema languages extended from these three languages, and schema languages equivalent in description ability to these three languages.

4. The structured data processing apparatus according to any one of claims 1 to 3, wherein
the schema format conversion instruction information and the schema resource definition information are data described in one of the XSL, the language extended from the XSL, and tree structure conversion languages equal in description ability to the XSL and the XSL extended language.

5. The structured data processing apparatus according to any one of claims 1 to 4, wherein
the structured data includes an element about at least one of sequence information, which includes one of or both of base sequences and amino acid sequences, and literature information.

6. A structured data processing method comprising:
a structure data acquisition step of acquiring structured data described in a structured description language, and schema data defining a structure of the structured data;
a format conversion step of converting the structured data and the schema data acquired by the structured data acquisition unit based on schema format conversion instruction information;
a structured data registration step of registering the structured data and the schema data converted by the format conversion unit in a database;
an analysis tool registration step of registering a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and
an analysis tool starting step of converting the structured data and the schema data registered in the database according to the schema resource definition information corresponding to the tool program, and inputting the converted structured data and schema data to the tool program if the tool program is started.

7. The structured data processing method according to claim 6, wherein
the structured description language is one of XML, SGML, BioML, BSML, ASN.1, GAME, structured description languages extended from these six languages, and structured description languages equivalent in description ability to these six languages.

8. The structured data processing method according to claim 6 or 7, wherein
the schema data is data described in one of DTD, XML schema, RELAX, schema languages extended from these three languages, and schema languages equivalent in description ability to these three languages.

9. The structured data processing method according to any one of claims 6 to 8, wherein
the schema format conversion instruction information and the schema resource definition information are data described in one of the XSL, the language extended from the XSL, and tree structure conversion languages equal in description ability to the XSL and the XSL extended language.

10. The structured data processing method according to any one of claims 6 to 9, wherein
the structured data includes an element about at least one of sequence information, which includes one of or both of base sequences and amino acid sequences, and literature information.

11. A program which allows a computer to execute a structured data processing method comprising:
a structure data acquisition step of acquiring structured data described in a structured description language, and schema data defining a structure of the structured data;
a format conversion step of converting the structured data and the schema data acquired by the structured data acquisition unit based on schema format conversion instruction information;
a structured data registration step of registering the structured data and the schema data converted by the format conversion unit in a database;
an analysis tool registration step of registering a tool program for accessing the database, in which the structured data and the schema data are registered by the structured data registration unit, to conduct a data processing, and schema resource definition information which defines resources of a schema of the structured data input to the tool program so that the tool program corresponds to the schema resource definition information; and
an analysis tool starting step of converting the structured data and the schema data registered in the database according to the schema resource definition information corresponding to the tool program, and inputting the converted structured data and schema data to the tool program if the tool program is started.

12. The program according to claim 11, wherein
the structured description language is one of XML, SGML, BioML, BSML, ASN.1, GAME, structured description languages extended from these six languages, and structured description languages equivalent in description ability to these six languages.

13. The program according to claim 11 or 12, wherein
the schema data is data described in one of DTD, XML schema, RELAX, schema languages extended from these three languages, and schema languages equivalent in description ability to these three languages.

14. The program according to any one of claims 11 to 13, wherein
the schema format conversion instruction information, and the schema resource definition information are data described in one of the XSL, the language extended from the XSL, and tree structure conversion languages equal in description ability to the XSL and the XSL extended language.

15. The program according to any one of claims 11 to 14, wherein
the structured data includes an element about at least one of sequence information, which includes one of or both of base sequences and amino acid sequences, and literature information.

16. A computer readable recording medium which records the program according to any one of claims 11 to 15.
